# EUROPEAN PATENT APPLICATION

(11) **EP 4 248 974 A1**
(43) Date of publication of application: **27.09.2023**
(21) Application number: 21894736.4
(22) Date of filing: 18.11.2021
(51) Int. Cl.: A61K 31/519, A61K 31/5377, A61K 31/541, A61P 25/00, A61P 35/00, A61P 35/04, C07D 487/04

(54) **BRAIN-MIGRATING TUMOR THERAPEUTIC AGENT CONTAINING FUSED PYRIMIDINE COMPOUND AS ACTIVE INGREDIENT**

(30) Priority: 20.11.2020 JP 2020193073
(71) Applicant: Taiho Pharmaceutical Co., Ltd., Chiyoda-ku, Tokyo 101-8444 (JP)
(72) Inventor: MIYAZAKI, Isao, Tsukuba-shi, Ibaraki 300-2611 (JP); IGUCHI, Satoru, Tsukuba-shi, Ibaraki 300-2611 (JP); WAKAYAMA, Kentaro, Tsukuba-shi, Ibaraki 300-2611 (JP)
(74) Representative: Hoffmann Eitle
(86) International application number: PCT/JP2021/042502
(87) International publication number: WO 2022/107864

(57) **Abstract**

An object to be solved of the present invention is to provide a brain-penetrable antitumor agent showing an excellent brain penetration property and RET inhibitory activity.

The present invention provides brain-penetrable antitumor agent including a compound represented by Formula (I) below or a salt thereof as an active ingredient: 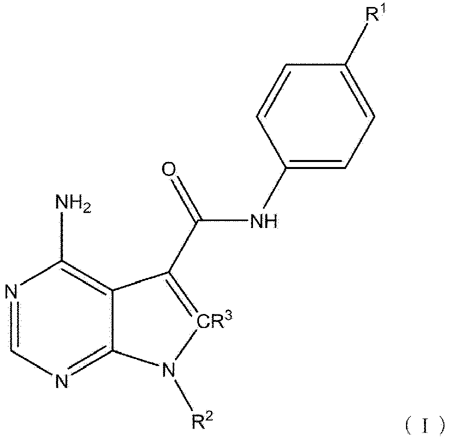 wherein R¹, R², and R³ are as described in Specification.

## Description

### [Technical Field]

### Cross-Reference to Related Applications

The present application claims priority to Japanese Patent Application No. 2020-193073 filed on November 20, 2020, the entire contents of which are incorporated herein by reference.

The present invention relates to an antitumor agent.

### [Background Art]

A brain tumor is a disease of the brain, and refers to a tumor that occurs in an intracranial tissue. Brain tumors are classified into a primary brain tumor, which occurs from brain cells or the like, and a metastatic brain tumor, which is formed by metastasis of cancer, such as lung cancer or breast cancer, to the brain. Symptomatic metastatic brain tumors have been reported to occur in 8 to 10% of cancer patients, and there is also a report that, in lung cancer, brain metastasis has been reported at a frequency of 40 to 50% according to autopsy (Non-patent Literature 1 to 3) . Known primary sites for metastatic brain tumors include lung cancer, breast cancer, gastrointestinal cancer (gastric cancer), malignant melanoma, renal and urinary cancer, etc., and of those, lung cancer accounts for about a half of brain metastases (Non-patent Literature 2). A treatment method for the metastatic brain tumor differs depending on a systemic condition, the size of the tumor, the number of metastases, etc. As the treatment method, radiation treatment, an operation, pharmacotherapy, or a combination thereof are performed. A surgical operation is generally selected in, for example, the following case: the primary site is under control, the brain tumor to be treated is solitary, and a certain period of survival is expected. However, a tumor that has metastasized into the brain is often entangled with the brain parenchyma and/or cranial nerve tissue in a complex manner, and it is often impossible to completely resect only the tumor by the surgical operation. For the radiation treatment, two treatment methods are known: quantitative radiosurgical treatment (e.g., gamma knife therapy) and whole brain irradiation involving irradiating the whole brain with a radiation. The treatment of the metastatic brain tumor is currently performed by a combination of the surgical operation and the radiation treatment in many cases, but it can hardly be said that a sufficient therapeutic effect is achieved (Non-patent Literature 4 and 5). In general, the central nervous system (CNS) including the brain is protected by a highly specialized layer of cells closely adhering to each other, called the blood-brain barrier (BBB), so that harmful substances may not enter. One reason that effective medicaments have yet to be developed for many CNS-related diseases is that almost all therapeutic molecules including antibodies cannot pass through the BBB. Low-molecular-weight compounds are no exception, and there is even a report that more than 98% of the low-molecular-weight compounds cannot pass through the BBB (Non-patent Literature 6) . At present, there are many compounds showing efficacy in peripheral cancers (primary sites), but their efficacy in brain metastatic lesions of the cancers is limited. One possible reason therefor is that those compounds cannot reach the inside of the brain in amounts sufficient for achieving their effects, thus failing to achieve the same effects as in the primary sites (Non-patent Literature 7 and 8) . Accordingly, in the CNS-related diseases and brain tumors, it is desired that a low-molecular-weight compound having a high brain penetration property and a brain penetration method therefor be developed. In particular, in the treatment of the metastatic brain tumor, there is a demand for a medicament that shows an effective effect on a primary site, such as lung cancer, and also shows a high CNS penetration property, thereby showing an effect on a brain metastatic lesion as well.

For example, a large number of compounds have shown efficacy in primary sites, and an example thereof is a compound having RET inhibitory activity. Here, RET is a receptor tyrosine kinase identified as one of the proto-oncogenes. RET binds to the glial cell line-derived neurotrophic factor (GDNF) and GDNF receptor to form a complex, which enables RET to perform physiological functions through intracellular phosphorylation signaling (Non-patent Literature 9) . A study reports that in normal tissues, RET contributes to kidney development and neurogenesis during fetal life (Non-patent Literature 10) . Some studies indicate that in cancers, such as lung cancer, thyroid cancer, breast cancer, pancreas cancer, and prostate cancer, the translocation, mutation, and overexpression of RET enhances its activation to thereby contribute to cell growth, tumor formation, or tissue infiltration (Non-patent Literature 11 to 16) . In addition, RET is known to be a poor prognostic factor of cancer, as indicated in some reports that the translocation of RET and its enhanced activation level are also inversely correlated with prognosis in cancer (Non-patent Literature 17 to 19).

Therefore, an inhibitor capable of inhibiting RET activity is thought to be useful as a therapeutic agent for diseases associated with abnormally enhanced RET signaling pathways.

It is expected, for example, that in cancers involving translocated, mutated, and overexpressed RET, the administration of a medicament capable of specifically inhibiting RET will selectively and intensively suppress the proliferation of cancer cells and contribute to the treatment, life prolongation, and improvement in quality of life of cancer patients.

As such compounds having RET inhibitory activity, well-known compounds are given, and for example, PP1 is known (Non-patent Literature 20). In PP1, a p-tolyl group is bonded to a fused ring pyrimidine skeleton. PP1 is known to exhibit high inhibitory activity against not only RET but also Src (Non-patent Literature 21), c-Kit, Bcr-Abl (Non-patent Literature 22 and 23), and others. For example, as side effects, the inhibition of Src may lead to abnormally enhanced bone formation, and the inhibition of Lck may suppress T cells (Non-patent Literature 24 and 25). Since multikinase inhibitors inhibit not only RET but also various signaling pathways to inhibit cell growth and other functions, the inhibitors raise concerns about possible various side effects, which may require dose reduction and/or drug holidays, thus leading to insufficient RET inhibitory activity. From the standpoint of side-effect reduction, RET inhibitors that have high inhibitory activity against RET with low inhibitory activity against other kinases have been desired. However, hitherto, there has been reported no medicament showing a potent RET inhibitory effect and also showing an excellent brain penetration property.

### [Citation List]

### [Non-patent Literature]

[Non-patent Literature 1] Qingbei Zeng, J Med Chem. 22; 58 (20) : 8200-15, (2015).
[Non-patent Literature 2] Lakshmi Nayak, Curr Oncol Rep; 14 (1) : 48-54, (2012)
[Non-patent Literature 3] Brunilde Gril, Eur J Cancer. ; 46(7) : 1204-10, (2010)
[Non-patent Literature 4] Taofeek K. Owonikoko, Nat Rev Clin Oncol.; 11(4): 203-22, (2014)
[Non-patent Literature 5] Chien-Hung Gow, Clin Cancer Res. 1; 14(1): 162-8, (2008)
[Non-patent Literature 6] William M. Pardridge, J Neurochem. 70(5): 1781-92, (1998)
[Non-patent Literature 7] Ted W. Johnson, J Med Chem. 12; 57(11): 4720-44, (2014)
[Non-patent Literature 8] Victor A. Levin, Neuro Oncol.; 17 Suppl 6: vi1-26 (2015)
[Non-patent Literature 9] Lois M. Mulligan, NatureRev., 14(3) : pp173-186, (2014)
[Non-patent Literature 10] Carlos F. Ibanez, Cold Spring Harb Perspect Biol., 5(2): pp1-10, (2013)
[Non-patent Literature 11] Takashi Kohno, Nature Med., 18 (3) : pp375-377, (2012)
[Non-patent Literature 12] Massimo Santoro, Eur J Endocrinol., 155: pp645-653, (2006)
[Non-patent Literature 13] Marjan Zarif Yeganeh, Asian Pac J Cancer Prev., 16(6): pp2107-2117, (2015)
[Non-patent Literature 14] Albana Gattelli, EMBO Mol Med., 5: pp1335-1350, (2013)
[Non-patent Literature 15] Yoshinori Ito, Surgery, 138: pp788-794, (2005)
[Non-patent Literature 16] DawnM. Dawson, J Natl Cancer Inst. , 90: pp519-523, (1998)
[Non-patent Literature 17] Weijing Cai, Cancer, 119: pp1486-1494, (2013)
[Non-patent Literature 18] Rossella Elisei, J Clin Endocrinol Metab., 93(3): pp682-687, (2008)
[Non-patent Literature 19] Q ZENG, J. Int. Med. Res., 36: pp656-664, (2008)
[Non-patent Literature 20] Francesca Carlomagno, Cancer Res., 62(4): pp1077-1082, (2002)
[Non-patent Literature 21] Johannes Waltenberger, Circ Res., 85(1): pp 12 - 2 2, (1999)
[Non-patent Literature 22] Louise Tatton, JBiolChem., 278(7) : pp4847-4853, (2003)
[Non-patent Literature 23] Markus Warmuth, Blood. 101(2): pp664-672, (2003)
[Non-patent Literature 24] Carolyn Lowe, Proc Natl Acad Sci USA, 90(10): pp4485-9, (1993)
[Non-patent Literature 25] ThierryMolina, Nature, 357(6374): pp161-4, (1992)

### [Summary of the Invention]

### [Technical Problem]

An object of the present invention is to provide a brain-penetrable antitumor agent showing an excellent brain penetration property and RET inhibitory activity. Another object of the present invention is to provide use of a compound or a salt thereof for producing a brain-penetrable antitumor agent showing an excellent brain penetration property and RET inhibitory and antitumor effects, or for treating a tumor through use of a brain-penetrable antitumor agent. Still another object of the present invention is to provide a method for treating a tumor, in particular, a brain tumor, comprising administering a brain-penetrable antitumor agent comprising a compound having RET inhibitory activity or a salt thereof.

### [Solution to Problem]

The present inventors conducted extensive research to achieve the above objects, and consequently found that a fused pyrimidine compound having a specific structure capable of selectively inhibiting RET or a salt thereof was useful as a brain-penetrable antitumor agent. Thus, the present invention has been completed.

Specifically, the present invention includes the following embodiments.

[1] A brain-penetrable antitumor agent comprising a compound represented by Formula (I) below or a salt thereof as an active ingredient: 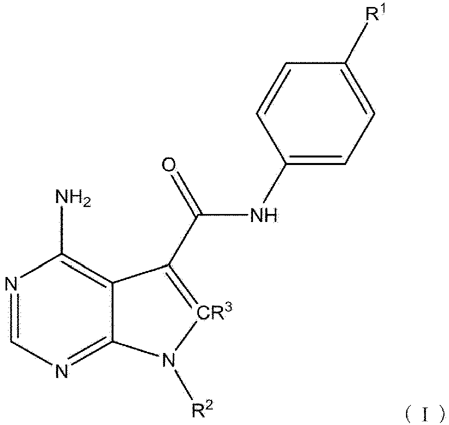 wherein
   R¹ is C1-C6 alkoxyalkyl,
   R² is substituted or unsubstituted C3-C5 cycloalkyl, and
   R³ is hydrogen,
   substituted or unsubstituted C2-C6 alkynyl, or
   substituted or unsubstituted C1-C6 alkoxy.
[2] The brain-penetrable antitumor agent according to [1], wherein the substituted or unsubstituted C3-C5 cycloalkyl represented by R² is substituted with:
   (1-1) C1-C2 alkyl.
[3] The brain-penetrable antitumor agent according to any one of [1] or [2], wherein R² is C3-C4 cycloalkyl that may be substituted with methyl.
[4] The brain-penetrable antitumor agent according to any one of [1] to [3], wherein the substituted or unsubstituted C2-C6 alkynyl represented by R³ is substituted with:
   (2-1) substituted or unsubstituted amino;
   (2-2) substituted or unsubstituted C1-C6 alkyl;
   (2-3) a substituted or unsubstituted 4- to 10-membered monocyclic saturated heterocyclic group containing 1 to 3 identical or different heteroatoms selected from nitrogen, oxygen, and sulfur; or
   (2-4) a substituted or unsubstituted 4- to 10-membered monocyclic unsaturated heterocyclic group containing 1 to 3 identical or different heteroatoms selected from nitrogen, oxygen, and sulfur.
[5] The brain-penetrable antitumor agent according to any one of [1] to [4], wherein the substituted or unsubstituted C1-C6 alkoxy represented by R³ is substituted with:
   (3-1) amino;
   (3-2) C1-C6 alkyl that may have hydroxy;
   (3-3) a 4- to 10-membered monocyclic saturated heterocyclic group that may be substituted with at least one kind selected from the group consisting of methyl, ethyl, and amino, and contains 1 to 3 identical or different heteroatoms selected from nitrogen, oxygen, and sulfur; or
   (3-4) a 4- to 10-membered monocyclic unsaturated heterocyclic group that may be substituted with at least one kind selected from the group consisting of methyl, ethyl, and amino, and contains 1 to 3 identical or different heteroatoms selected from nitrogen, oxygen, and sulfur.
[6] The antitumor agent according to any one of [1] to [5], wherein
   R¹ is C1-C6 alkoxy C1-C6 alkyl,
   R² is C3-C5 cycloalkyl that may be substituted with C1-C2 alkyl, and
   R³ is
   substituted or unsubstituted C2-C6 alkynyl; or
   substituted or unsubstituted C1-C6 alkoxy,

   wherein the substituted or unsubstituted C2-C6 alkynyl represented by R³ is substituted with:
      amino;
      C1-C6 alkyl that may be substituted with at least one kind selected from the group consisting of hydroxy, amino, and cyano;
      a 4- to 10-membered monocyclic saturated heterocyclic group that may be substituted with at least one kind selected from the group consisting of C1-C6 alkyl, hydroxy, amino, and cyano, and contains 1 to 3 identical or different heteroatoms selected from nitrogen, oxygen, and sulfur; or
      a 4- to 10-membered monocyclic unsaturated heterocyclic group that may be substituted with at least one kind selected from the group consisting of C1-C6 alkyl, hydroxy, amino, and cyano, and contains 1 to 3 identical or different heteroatoms selected from nitrogen, oxygen, and sulfur, and
   the substituted or unsubstituted C1-C6 alkoxy represented by R³ is substituted with:
      amino;
      C1-C6 alkyl that may have hydroxy;
      a 4- to 10-membered monocyclic saturated heterocyclic group that may be substituted with at least one kind selected from the group consisting of methyl, ethyl, and amino, and contains 1 to 3 identical or different heteroatoms selected from nitrogen, oxygen, and sulfur; or
      a 4- to 10-membered monocyclic unsaturated heterocyclic group that may be substituted with at least one kind selected from the group consisting of methyl, ethyl, and amino, and contains 1 to 3 identical or different heteroatoms selected from nitrogen, oxygen, and sulfur.
[7] The brain-penetrable antitumor agent according to any one of [1] to [6], wherein the compound represented by Formula (I) or a salt thereof is any of the following or a salt thereof:
   (1) 4-amino-6-[2-(1,3-dimethyl-1H-pyrazol-4-yl)ethynyl] -N-[4-(methoxymethyl)phenyl]-7-(1-methylcyclopropyl)-7H-pyrrol o[2,3-d]pyrimidine-5-carboxamide;
   (2) 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-car boxamide;
   (3) 4-amino-N-[4-(methoxymethyl)phenyl]-7-(1-methylcyclopropyl)-6-[(1-methylpiperidin-4-yl)ethynyl]-7H-pyrrolo[2,3-d]pyrimidine-5-carboxamide;
   (4) 4-amino-N-[4-(methoxymethyl)phenyl]-6-((1-methyl-1H-pyrazol-4-yl)ethynyl)-7-(1-methylcyclopropyl)-7H-pyrrolo[2,3-d]pyrimidin e-5-carboxamide;
   (5) 4-amino-N-[4-(methoxymethyl)phenyl]-7-(1-methylcyclopropyl)-6-(prop-1-yn-1-yl)-7H-pyrrolo[2,3-d]pyrimidine-5-carboxamide;
   (6) 4-amino-6-[3-(dimethylamino)prop-1-yn-1-yl]-N-[4-(methoxymethyl)phenyl]-7-(1-methylcyclopropyl)-7H-pyrrolo[2 ,3-d]pyrimidine-5-carboxamide;
   (7) (R)-4-amino-N-[4-(methoxymethyl)phenyl]-7-(1-methylcyclopropyl )-6-((tetrahydrofuran-2-yl)methoxy)-7H-pyrrolo[2,3-d]pyrimidin e-5-carboxamide;
   (8) 4-amino-N-[4-(methoxymethyl)phenyl]-7-(1-methylcyclopropyl)-7H -pyrrolo[2,3-d]pyrimidine-5-carboxamide;
   (9) 4-amino-6-ethoxy-N-[4-(methoxymethyl)phenyl]-7-(1-methylcyclop ropyl)-7H-pyrrolo[2,3-d]pyrimidine-5-carboxamide;
   (10) 4-amino-N-(4-(methoxymethyl)phenyl)-6-((1-methyl-1H-imidazol-5 -yl)ethynyl)-7-(1-methylcyclopropyl)-7H-pyrrolo[2,3-d]pyrimidi ne-5-carboxamide;
   (11) 4-amino-N-[4-(methoxymethyl)phenyl]-7-(1-methylcyclopropyl)-6-(3-(piperidin-1-yl)prop-1-yn-1-yl)-7H-pyrrolo[2,3-d]pyrimidine -5-carboxamide;
   (12) 4-amino-N-[4-(methoxymethyl)phenyl]-7-(1-methylcyclopropyl)-6-(3-(pyrrolidin-1-yl)prop-1-yn-1-yl)-7H-pyrrolo[2,3-d]pyrimidin e-5-carboxamide;
   (13) 4-amino-N-[4-(methoxymethyl)phenyl]-7-(1-methylcyclopropyl)-6-((tetrahydro-2H-pyran-4-yl)ethynyl)-7H-pyrrolo[2,3-d]pyrimidin e-5-carboxamide;
   (14) 4-amino-6-(4-hydroxy-4-methylpent-1-yn-1-yl)-N-[4-(methoxymeth yl)phenyl]-7-(1-methylcyclopropyl)-7H-pyrrolo[2,3-d]pyrimidine -5-carboxamide;
   (15) 4-amino-N-[4-(methoxymethyl)phenyl]-7-(1-methylcyclopropyl)-6-[3-(tetrahydro-2H-pyran-4-yl)prop-1-yn-1-yl]-7H-pyrrolo[2,3-d] pyrimidine-5-carboxamide;
   (16) 4-amino-N-[4-(methoxymethyl)phenyl]-7-(1-methylcyclopropyl)-6-(pyridin-3-ylethynyl)-7H-pyrrolo[2,3-d]pyrimidine-5-carboxamid e;
   (17) 4-amino-6-[(6-aminopyridin-3-yl)ethynyl]-N-[4-(methoxymethyl)phenyl]-7-(1-methylcyclopropyl)-7H-pyrrolo[2 ,3-d]pyrimidine-5-carboxamide; and
   (18) 4-amino-N-[4-(methoxymethyl)phenyl]-7-(1-methylcyclopropyl)-6-(3-thiomorpholinoprop-1-yn-1-yl)-7H-pyrrolo[2,3-d]pyrimidine-5 -carboxamide.
[8] The brain-penetrable antitumor agent according to any one of [1] to [7], wherein the compound represented by Formula (I) or a salt thereof is any of the following or a salt thereof:
   (1) 4-amino-6-[2-(1,3-dimethyl-1H-pyrazol-4-yl)ethynyl] -N-[4-(methoxymethyl)phenyl]-7-(1-methylcyclopropyl)-7H-pyrrol o[2,3-d]pyrimidine-5-carboxamide;
   (2) 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-car boxamide;
   (4) 4-amino-N-[4-(methoxymethyl)phenyl]-6-((1-methyl-1H-pyrazol-4-yl)ethynyl)-7-(1-methylcyclopropyl)-7H-pyrrolo[2,3-d]pyrimidin e-5-carboxamide;
   (5) 4-amino-N-[4-(methoxymethyl)phenyl]-7-(1-methylcyclopropyl)-6-(prop-1-yn-1-yl)-7H-pyrrolo[2,3-d]pyrimidine-5-carboxamide;
   (6) 4-amino-6-[3-(dimethylamino)prop-1-yn-1-yl]-N-[4-(methoxymethyl)phenyl]-7-(1-methylcyclopropyl)-7H-pyrrolo[2 ,3-d]pyrimidine-5-carboxamide;
   (9) 4-amino-6-ethoxy-N-[4-(methoxymethyl)phenyl]-7-(1-methylcyclop ropyl)-7H-pyrrolo[2,3-d]pyrimidine-5-carboxamide;
   (12) 4-amino-N-[4-(methoxymethyl)phenyl]-7-(1-methylcyclopropyl)-6-(3-(pyrrolidin-1-yl)prop-1-yn-1-yl)-7H-pyrrolo[2,3-d]pyrimidin e-5-carboxamide;
   (13) 4-amino-N-[4-(methoxymethyl)phenyl]-7-(1-methylcyclopropyl)-6-((tetrahydro-2H-pyran-4-yl)ethynyl)-7H-pyrrolo[2,3-d]pyrimidin e-5-carboxamide; and
   (16) 4-amino-N-[4-(methoxymethyl)phenyl]-7-(1-methylcyclopropyl)-6-(pyridin-3-ylethynyl)-7H-pyrrolo[2,3-d]pyrimidine-5-carboxamid e.
[9] The brain-penetrable antitumor agent according to any one of [1] to [8], for treating a primary or metastatic brain tumor.
[10] Use of a compound represented by Formula (I) below or a salt thereof for producing a brain-penetrable antitumor agent: 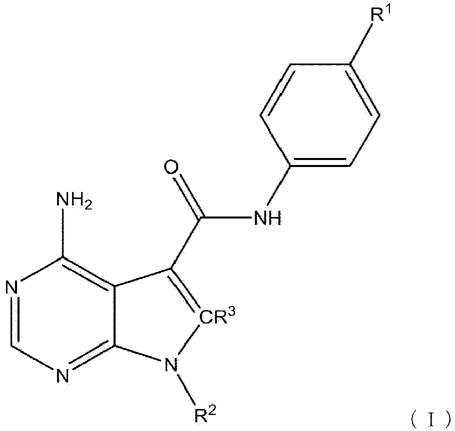 wherein
   R¹ is C1-C6 alkoxyalkyl,
   R² is substituted or unsubstituted C3-C5 cycloalkyl, and
   R³ is hydrogen,
   substituted or unsubstituted C2-C6 alkynyl, or
   substituted or unsubstituted C1-C6 alkoxy.
[11] A method for treating a subject having a tumor, comprising administering a brain-penetrable antitumor agent comprising an effective amount of a compound represented by Formula (I) below or a salt thereof to the subject in need of treatment: 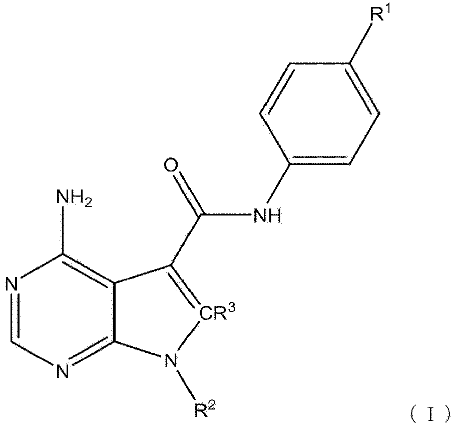 wherein
   R¹ is C1-C6 alkoxyalkyl,
   R² is substituted or unsubstituted C3-C5 cycloalkyl, and
   R³ is hydrogen,
   substituted or unsubstituted C2-C6 alkynyl, or
   substituted or unsubstituted C1-C6 alkoxy.
[12] A compound represented by Formula (I) below or a salt thereof for use in treatment of a tumor through use of a brain-penetrable antitumor agent: 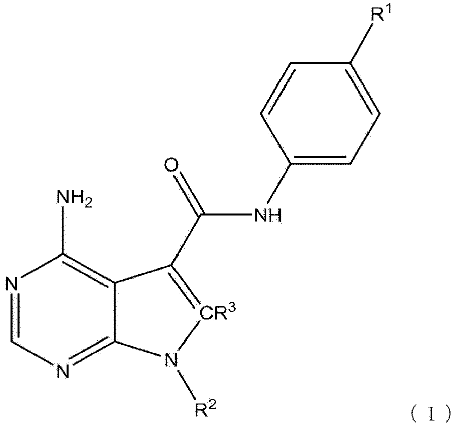 wherein
   R¹ is C1-C6 alkoxyalkyl,
   R² is substituted or unsubstituted C3-C5 cycloalkyl, and
   R³ is hydrogen,
   substituted or unsubstituted C2-C6 alkynyl, or
   substituted or unsubstituted C1-C6 alkoxy.
[13] Use of a compound represented by Formula (I) below or a salt thereof for treating a tumor through use of a brain-penetrable antitumor agent: 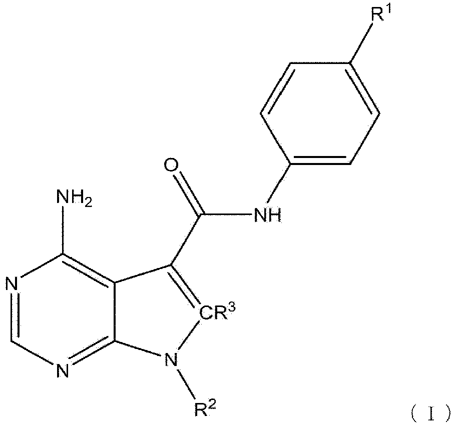 wherein
   R¹ is C1-C6 alkoxyalkyl,
   R² is substituted or unsubstituted C3-C5 cycloalkyl, and
   R³ is hydrogen,
   substituted or unsubstituted C2-C6 alkynyl, or
   substituted or unsubstituted C1-C6 alkoxy.
[14] A commercial package comprising:
   a compound represented by Formula (I) below or a salt thereof serving as an active ingredient: 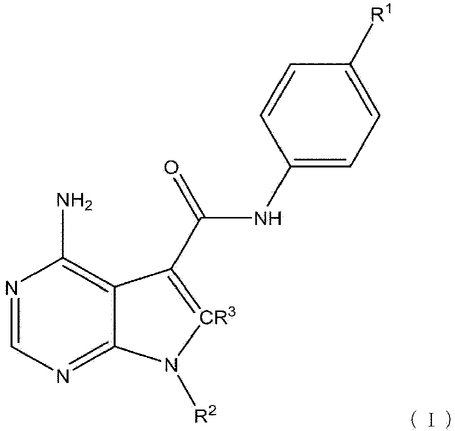 wherein
   R¹ is C1-C6 alkoxyalkyl,
   R² is substituted or unsubstituted C3-C5 cycloalkyl, and
   R³ is hydrogen,
   substituted or unsubstituted C2-C6 alkynyl, or
   substituted or unsubstituted C1-C6 alkoxy; and
   an instruction manual for use thereof for treating a tumor in a subject through use of a brain-penetrable antitumor agent.
[15] A brain-penetrable antitumor agent for treating a tumor with enhanced activation of RET, comprising a compound represented by Formula (I) below or a salt thereof as an active ingredient: 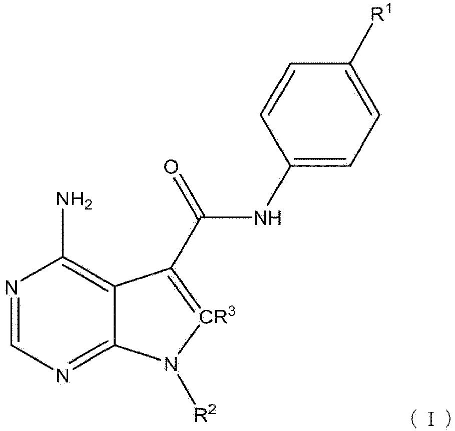 wherein
   R¹ is C1-C6 alkoxyalkyl,
   R² is substituted or unsubstituted C3-C5 cycloalkyl, and
   R³ is hydrogen,
   substituted or unsubstituted C2-C6 alkynyl, or
   substituted or unsubstituted C1-C6 alkoxy.
[16] Use of a compound represented by Formula (I) below or a salt thereof for producing a brain-penetrable antitumor agent for treating a tumor with enhanced activation of RET: 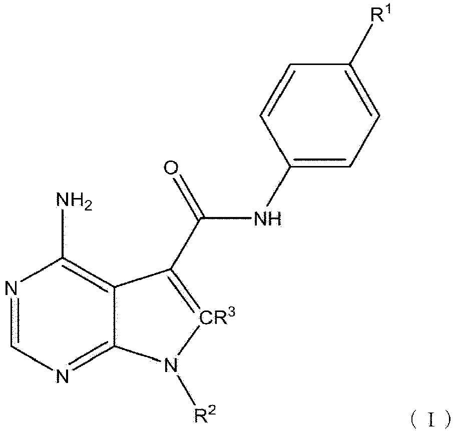 wherein
   R¹ is C1-C6 alkoxyalkyl,
   R² is substituted or unsubstituted C3-C5 cycloalkyl, and
   R³ is hydrogen,
   substituted or unsubstituted C2-C6 alkynyl, or
   substituted or unsubstituted C1-C6 alkoxy.
[17] A method for treating a subject having a tumor with enhanced activation of RET, comprising administering a brain-penetrable antitumor agent comprising an effective amount of a compound represented by Formula (I) below or a salt thereof to the subject: 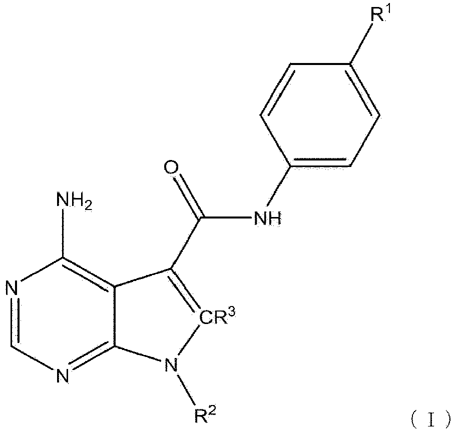 wherein
   R¹ is C1-C6 alkoxyalkyl,
   R² is substituted or unsubstituted C3-C5 cycloalkyl, and
   R³ is hydrogen,
   substituted or unsubstituted C2-C6 alkynyl, or
   substituted or unsubstituted C1-C6 alkoxy.
[18] A compound represented by Formula (I) below or a salt thereof for use in treatment of a tumor with enhanced activation of RET through use of a brain-penetrable antitumor agent: 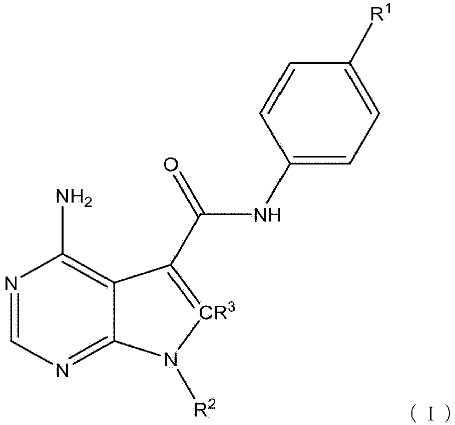 wherein
   R¹ is C1-C6 alkoxyalkyl,
   R² is substituted or unsubstituted C3-C5 cycloalkyl, and
   R³ is hydrogen,
   substituted or unsubstituted C2-C6 alkynyl, or
   substituted or unsubstituted C1-C6 alkoxy.
[19] Use of a compound represented by Formula (I) below or a salt thereof for treating a tumor with enhanced activation of RET through use of a brain-penetrable antitumor agent: 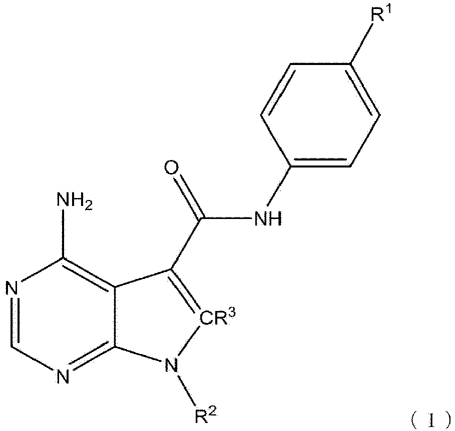 wherein
   R¹ is C1-C6 alkoxyalkyl,
   R² is substituted or unsubstituted C3-C5 cycloalkyl, and
   R³ is hydrogen,
   substituted or unsubstituted C2-C6 alkynyl, or
   substituted or unsubstituted C1-C6 alkoxy.
[20] A commercial package comprising:
   a compound represented by Formula (I) below or a salt thereof serving as an active ingredient: 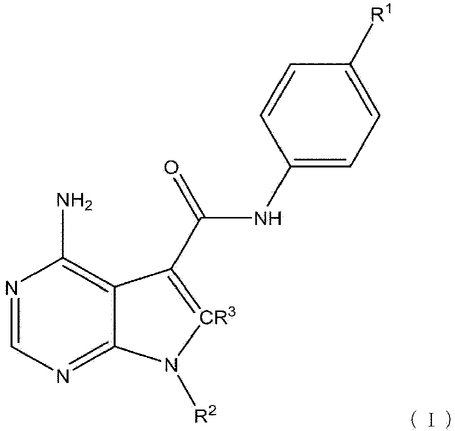 wherein
   R¹ is C1-C6 alkoxyalkyl,
   R² is substituted or unsubstituted C3-C5 cycloalkyl, and
   R³ is hydrogen,
   substituted or unsubstituted C2-C6 alkynyl, or
   substituted or unsubstituted C1-C6 alkoxy; and
   an instruction manual for use thereof for treating a tumor with enhanced activation of RET in a subject through use of a brain-penetrable antitumor agent.

### [Advantageous Effects of Invention]

The present invention provides an antitumor agent having a brain penetration property. A preferred mode of the present invention provides a novel therapeutic agent having RET inhibitory activity with an effective therapeutic effect on a brain tumor. Further, the brain-penetrable antitumor agent of the present invention has highly selective inhibitory activity on RET, and hence is useful because toxicity caused by the inhibition of any other kinase is expected to be reduced and a high therapeutic effect is expected.

### [Brief Description of Drawing]

Fig. 1 shows the antitumor effect of the compound of the present invention in a brain implantation model animal (NIH/3T3_CCDC6-RET brain transplant model).
Fig. 2 shows the rate of change in body weight of the brain implantation model animal (NIH/3T3_CCDC6-RET brain transplant model) by the compound of the present invention.

### [Description of Embodiments]

One mode of the present invention relates to a brain-penetrable antitumor agent comprising a compound represented by Formula (I) below or a salt thereof as an active ingredient.

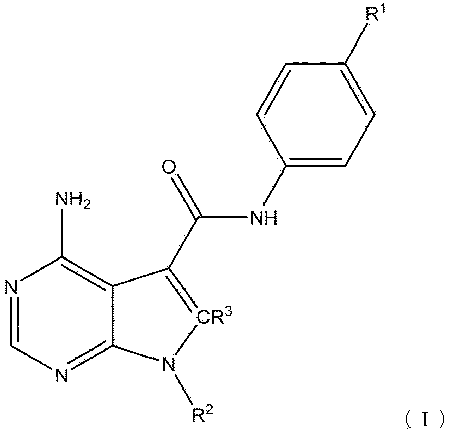

In the present invention, the compound represented by Formula (I) is sometimes simply referred to as compound (I).

In the present invention, the term "brain-penetrable antitumor agent" refers to an antitumor agent intended to allow at least part of its active ingredient to penetrate to the brain to achieve a therapeutic effect.

In the present specification, unless otherwise specified, examples of the "substituent" include deuterium, halogen, hydroxy, alkyl, alkoxy, alkoxyalkyl, cycloalkyl, cycloalkyl-alkyl, alkenyl, alkynyl, amino, mono-or dialkylamino, oxo, saturated or unsaturated heterocyclic group (the saturated or unsaturated heterocyclic group may have at least one selected from the group consisting of C1-C6 alkyl, hydroxy, amino and cyano as substituent(s)), aromatic hydrocarbon, etc. When a substituent listed above is present, the number thereof is not limited, but typically one, two, or three. In the present specification, when there are several substituents, the substituents may be the same or different, unless otherwise specified.

In the present specification, when there are several options for the substituents possessed by each group defined in Formula (I), each group may have the same or different types of substituents, unless otherwise specified. For example, "alkyl that is substituted with halogen or hydroxy" includes not only alkyl that is substituted with halogen alone and alkyl that is substituted with hydroxy alone, but also alkyl that is substituted with both halogen and hydroxy, unless otherwise specified. Moreover, "alkyl that is substituted with halogen or hydroxy" includes, for example, alkyl that is substituted with two or more kinds of halogen atoms (e.g., fluorine and chlorine) .

In the present specification, examples of the "halogen" include fluorine, chlorine, bromine, and iodine.

In the present specification, the "alkyl" refers to linear or branched saturated hydrocarbon. Examples include methyl, ethyl, n-propyl, isopropyl, n-butyl, sec-butyl, isobutyl, tert-butyl, 1-methylpropyl, n-pentyl, isopentyl, tert-pentyl, pentan-3-yl, n-hexyl, 1,1-dimethylpropyl, 1,1,2,2-tetramethylethyl, n-heptyl, 1,1,2,2-tetramethylpropyl, n-octyl, n-nonyl, n-decyl, etc.; and specifically include C1-C10 alkyl, C3-C10 alkyl, C1-C6 alkyl, C1-C4 alkyl, C3-C8 alkyl, C3-C6 alkyl, etc.

In the present specification, the "alkoxy" refers to oxy to which alkyl mentioned above is bonded. Examples include methoxy, ethoxy, n-propoxy, isopropoxy, n-butoxy, isobutoxy, tert-butoxy, n-pentyloxy, isopentyloxy, n-hexyloxy, etc.; and specifically include C1-C6 alkoxy, C1-C4 alkoxy, etc.

In the present specification, the "alkoxyalkyl" refers to alkyl mentioned above having one or more (e.g., 1 to 5, preferably 1 to 3, and more preferably 1) alkoxy groups mentioned above. Examples include methoxymethyl, ethoxymethyl, n-propoxymethyl, n-butoxymethyl, 2-methoxyethyl, 1-methoxy-n-propyl, 3-methoxy-n-propyl, 2-ethoxy-n-butyl, 4-methoxy-n-butyl, 5-methoxy-n-pentyl, 6-methoxy-n-hexyl, etc.; and specifically include C1-C6 alkoxy C1-C6 alkyl, C1-C4 alkoxy C1-C6 alkyl, C1-C4 alkoxy C1-C4 alkyl, etc.

In the present specification, the "C1-C6 alkoxyalkyl" refers to alkyl to which C1-C6 alkoxy mentioned above is bonded. Examples include methoxymethyl, ethoxymethyl, n-propoxymethyl, n-butoxymethyl, 2-methoxyethyl, 1-methoxy-n-propyl, 3-methoxy-n-propyl, 2-ethoxy-n-butyl, 4-methoxy-n-butyl, 5-methoxy-n-pentyl, 6-methoxy-n-hexyl, 8-methoxy-n-octyl, etc.

In the present specification, the "cycloalkyl" refers to monocyclic or polycyclic (e.g., bicyclic or tricyclic) saturated hydrocarbon. Examples include monocyclic cycloalkyl, such as cyclopropyl, cyclobutyl, cyclopentyl, cyclohexyl, and cycloheptyl; polycyclic cycloalkyl, such as spiro[3.3]heptyl, spiro[3.4]octyl, and dispiro [5.1.78.26]heptadecanyl; and specifically include C3-C7 cycloalkyl, C3-C5 cycloalkyl, etc.

In the present specification, the "aromatic hydrocarbon" refers to a monocyclic or polycyclic (e.g., bicyclic or tricyclic) ring substituent comprising carbon and hydrogen having an unsaturated bond, and containing 4e+2 number of electrons (e is an integer of 1 or more) in the cyclic π electron system. Examples include phenyl, naphthyl, anthracenyl, phenanthryl, fluorenyl, tetrahydronaphthyl, etc.; and specifically include C6-C14, C6-C10, and C8-C14 aromatic hydrocarbons.

In the present specification, the "alkenyl" refers to linear or branched unsaturated hydrocarbon having at least one (e.g., 1 or 2, or 1) double bond. Examples include vinyl, allyl, 1-propenyl, 2-methyl-2-propenyl, isopropenyl, 1-, 2- or 3-butenyl, 2-, 3-, or 4-pentenyl, 2-methyl-2-butenyl, 3-methyl-2-butenyl, 5-hexenyl, 3-methyl-3-butenyl, etc.; and specifically include C2-C6 alkenyl, C2-C4 alkenyl, etc.

In the present specification, the "alkynyl" refers to linear or branched unsaturated hydrocarbon having at least one (e.g., 1 or 2, or 1) triple bond. Examples include ethynyl, 1- or 2-propynyl, 1-, 2-, or 3-butynyl, 1-methyl-2-propynyl, etc.; and specifically include C2-C6 alkynyl, C2-C4 alkynyl, etc.

In the present specification, the "saturated heterocyclic group" refers to a monocyclic or polycyclic (e.g., bicyclic or tricyclic) saturated heterocyclic group having one or more (e.g., 1 to 3) identical or different heteroatoms selected from nitrogen, oxygen, and sulfur. Examples include morpholino, 1-pyrrolidinyl, 3-pyrrolidinyl, piperidino, piperazinyl, 4-methyl-1-piperazinyl, tetrahydrofuranyl, tetrahydropyranyl, tetrahydrothiophenyl, thiazolidinyl, oxazolidinyl, thiomorpholinyl, 7-azabicyclo[2.2.1]hept-2-yl, 2,6-dioxabicyclo[3.2.1]oct-7-yl, 7-oxabicyclo[2.2.1]heptane, etc.; and specifically include 4- to 6-membered, 4- to 10-membered, 8- to 14-membered, and 8- to 10-membered saturated heterocyclic groups.

In the present specification, the "unsaturated heterocyclic group" refers to a monocyclic or polycyclic (e.g., bicyclic or tricyclic), completely or partially unsaturated heterocyclic group having one or more (e.g., 1 to 3) identical or different heteroatoms selected from nitrogen, oxygen, and sulfur. Examples include imidazolyl, thienyl, furyl, pyrrolyl, oxazolyl, isoxazolyl, thiazolyl, isothiazolyl, thiadiazolyl, pyrazolyl, triazolyl, tetrazolyl, pyridinyl, pyrazyl, pyrimidinyl, pyridazinyl, indolyl, isoindolyl, indazolyl, triazolopyridinyl, benzoimidazolyl, benzoxazolyl, benzothiazolyl, benzothienyl, benzofuranyl, purinyl, quinolyl, isoquinolyl, quinazolinyl, quinoxalinyl, methylenedioxyphenyl, ethylenedioxyphenyl, dihydrobenzofuranyl, dihydrothiazolyl, benzothiophenyl, etc.; and specifically include 4- to 6-membered, 4- to 10-membered, 8- to 14-membered, and 8- to 10-membered unsaturated heterocyclic groups.

The term "Ca-Cb" in the description regarding the substituent in the present specification indicates that the substituent has a- to b-number of carbon atoms. For example, "C1-C6 alkyl" refers to alkyl having 1 to 6 carbon atoms, and "C6-C14 aromatic hydrocarbon oxy" refers to oxy to which aromatic hydrocarbon having 6 to 14 carbon atoms is bonded. Further, the term "a- to b-membered" indicates that the number of atoms (number of ring members) that constitute the ring is a to b. For example, a "4- to 10-membered saturated heterocyclic group" refers to a saturated heterocyclic group with a 4- to 10-membered ring.

Specific examples of the compound to be used as the active ingredient of the brain-penetrable antitumor agent of the present invention include, but are not limited to, those described below. The substituents, such as R¹, R², and R³, in the formula representing the compound (I) are specifically described below. In the description of the substituents, the substituents, such as R¹, R², and R³, refer to those substituents in Formula (I), unless otherwise specified.

R¹ is C1-C6 alkoxyalkyl.

Examples of the "C1-C6 alkoxyalkyl" represented by R¹ include those mentioned above, preferably C1-C4 alkoxyalkyl, and more preferably methoxymethyl.

Examples of the "substituent" in the "substituted or unsubstituted C3-C5 cycloalkyl" represented by R² (in the present specification, the "substituent" in the "substituted or unsubstituted C3-C5 cycloalkyl" represented by R² is sometimes referred to as "substituent R^{A}") include those mentioned above as examples of the "substituent," preferably C1-C6 alkyl, more preferably C1-C3 alkyl, and even more preferably methyl.

Examples of the "substituted or unsubstituted C3-C5 cycloalkyl" represented by R² include the C3-C5 cycloalkyl that has or does not have the substituent R^{A}, preferably C3-C4 cycloalkyl, and more preferably propyl.

Examples of the "C2-C6 alkynyl" in the "substituted or unsubstituted C2-C6 alkynyl" represented by R³ include those mentioned above;
preferably C2-C4 alkynyl; and
more preferably ethynyl or propynyl.

The number of triple bonds in the "C2-C6 alkynyl" in the "substituted or unsubstituted C2-C6 alkynyl" is preferably 1, and the position of the triple bond is preferably disposed between the carbon atom bonded to the 7H-pyrrolo [2,3-d] pyrimidine skeleton and a carbon atom adjacent to the carbon atom.

Preferred examples of the "substituent" in the "substituted or unsubstituted C2-C6 alkynyl" represented by R³ (in the present specification, the "substituent" in the "substituted or unsubstituted C2-C6 alkynyl" represented by R³ is sometimes referred to as "substituent R^{B}") include:
substituted or unsubstituted amino;
substituted or unsubstituted C1-C6 alkyl;
a substituted or unsubstituted 4- to 10-membered monocyclic saturated heterocyclic group containing 1 to 3 identical or different heteroatoms selected from nitrogen, oxygen, and sulfur;
a substituted or unsubstituted 4- to 10-membered monocyclic unsaturated heterocyclic group containing 1 to 3 identical or different heteroatoms selected from nitrogen, oxygen, and sulfur;
and the like.

The "substituent R^{B}" is more preferably
amino,
substituted or unsubstituted linear or branched C1-C6 alkyl,
a substituted or unsubstituted 4- to 10-membered monocyclic saturated heterocyclic group containing 1 to 3 identical or different heteroatoms selected from nitrogen, oxygen, and sulfur, or
a substituted or unsubstituted 4- to 10-membered monocyclic or polycyclic unsaturated heterocyclic group containing 1 to 3 identical or different heteroatoms selected nitrogen, oxygen, and sulfur, and
   even more preferably
amino;
C1-C6 alkyl that may be substituted with at least one kind selected from the group consisting of hydroxy, amino, and cyano;
a 4- to 10-membered monocyclic saturated heterocyclic group that may be substituted with at least one kind selected from the group consisting of C1-C6 alkyl, hydroxy, amino, and cyano, and contains 1 to 3 identical or different heteroatoms selected from nitrogen, oxygen, and sulfur; or
a 4- to 10-membered monocyclic unsaturated heterocyclic group that may be substituted with at least one kind selected from the group consisting of C1-C6 alkyl, hydroxy, amino, and cyano, and contains 1 to 3 identical or different heteroatoms selected from nitrogen, oxygen, and sulfur.

The "substituent R^{B}" is still more preferably
amino;
C1-C6 alkyl that may be substituted with hydroxy;
a 5- or 6-membered monocyclic saturated heterocyclic group that may be substituted with C1-C6 alkyl and contains 1 or 2 heteroatoms of nitrogen; or
a 5- or 6-membered monocyclic unsaturated heterocyclic group that may be substituted with at least one kind selected from the group consisting of C1-C6 alkyl and amino, and contains 1 or 2 identical or different heteroatoms selected from nitrogen, oxygen, and sulfur.

In the "substituted or unsubstituted amino" mentioned above as an example of the substituent (substituent R^{B}) in the "substituted orunsubstitutedC2-C6 alkynyl"representedby R³, preferred examples of the "substituent" include C1-C6 alkyl, hydroxy, and the like.

In the "substituted or unsubstituted C1-C6 alkyl" mentioned above as an example of the substituent (substituent R^{B}) in the "substituted or unsubstituted C2-C6 alkynyl" represented by R³, preferred examples of the "substituent" include hydroxy, C1-C6 alkoxy, and oxo, and preferably hydroxy.

In the "substituted or unsubstituted linear or branched C1-C6 alkyl" mentioned above as an example of the substituent (substituent R^{B}) in the "substituted or unsubstituted C2-C6 alkynyl" represented by R³, the "C1-C6 alkyl" is preferably branched C1-C6 alkyl, and more preferably branched C3-C6 alkyl.

Examples of the "substituted or unsubstituted C1-C6 alkyl" mentioned above as an example of the substituent (substituent R^{B}) in the "substituted or unsubstituted C2-C6 alkynyl" represented by R³ include preferably hydroxybutyl.

Preferred examples of the "substituent" of the "substituted or unsubstituted 4- to 10-memberedmonocyclic saturated heterocyclic group containing 1 to 3 identical or different heteroatoms selected from nitrogen, oxygen, and sulfur" mentioned above as an example of the substituent (substituent R^{B}) in the "substituted or unsubstituted C2-C6 alkynyl" represented by R³ include C1-C6 alkyl, hydroxy, amino, and cyano, preferably C1-C6 alkyl, and more preferably methyl.

The "substitutedor unsubstituted 4- to 10-membered monocyclic saturated heterocyclic group containing 1 to 3 identical or different heteroatoms selected from nitrogen, oxygen, and sulfur" mentioned above as an example of the substituent (substituent R^{B}) in the "substituted or unsubstituted C2-C6 alkynyl" represented by R³ is
preferably a "substituted or unsubstituted 4- to 6-membered monocyclic saturated heterocyclic group containing 1 or 2 identical or different heteroatoms selected from nitrogen, oxygen, and sulfur,"
more preferably a "substituted or unsubstituted 4- to 6-membered monocyclic saturated heterocyclic group containing 1 or 2 heteroatoms of nitrogen" (when the group has a substituent, the substituent is methyl),
even more preferably substituted or unsubstituted morpholino, tetrahydrofuranyl, tetrahydropyranyl, piperazinyl, pyrrolidinyl, piperidinyl, thiomorpholinyl, or oxetanyl (when the group has a substituent, the substituent is methyl, ethyl, or amino), and
still more preferably substituted or unsubstituted morpholino, tetrahydrofuranyl, tetrahydropyranyl, piperazinyl, pyrrolidinyl, piperidinyl, or thiomorpholinyl (when the group has a substituent, the substituent is preferably methyl or ethyl, and more preferably methyl).

In the "substituted or unsubstituted 4- to 10-membered monocyclic saturated heterocyclic group containing 1 to 3 identical or different heteroatoms selected from nitrogen, oxygen, and sulfur" mentioned above as an example of the substituent R^{B}, the number of substituents is not particularly limited, but is preferably 0 to 3, and more preferably 0 to 2.

Preferred examples of the "substituent" of the "substituted or unsubstituted 4- to 10-membered monocyclic unsaturated heterocyclic group containing 1 to 3 identical or different heteroatoms selected from nitrogen, oxygen, and sulfur" mentioned above as an example of the substituent (substituent R^{B}) in the "substituted or unsubstituted C2-C6 alkynyl" represented by R³ include C1-C6 alkyl, amino, hydroxy, and cyano, preferably C1-C6 alkyl or amino, and even more preferably methyl or amino.

The "substituted or unsubstituted 4- to 10-memberedmonocyclic unsaturated heterocyclic group containing 1 to 3 identical or different heteroatoms selected from nitrogen, oxygen, and sulfur" is
preferably a "substituted or unsubstituted 4- to 10-membered monocyclic unsaturated heterocyclic group containing 1 to 3 identical or different heteroatoms selected from nitrogen, oxygen, and sulfur" (the substituent thereof is C1-C6 alkyl, C1-C6 alkoxy, or amino),
more preferably a "substituted or unsubstituted 4- to 6-membered monocyclic unsaturated heterocyclic group containing 1 to 3 heteroatoms of nitrogen" (the substituent thereof is C1-C6 alkyl or amino),
even more preferably substituted or unsubstituted pyrazolyl, imidazo[1,2-b]pyridazinyl, imidazolyl, pyridinyl, thiazolyl, or furo[3,2-b]pyridinyl (the substituent thereof is methyl, ethyl, or amino), and
still more preferably substituted or unsubstituted pyrazolyl, imidazolyl, or pyridinyl (the substituent thereof is methyl, ethyl, or amino, and preferably methyl or amino).

In the "substituted or unsubstituted 4- to 10-membered monocyclic unsaturated heterocyclic group containing 1 to 3 identical or different heteroatoms selected from nitrogen, oxygen, and sulfur" mentioned above as an example of the substituent R^{B}, the number of substituents is not particularly limited, but is preferably 0 to 3, and more preferably 0 to 2.

In the present specification, the "substituent" in the "substituted or unsubstituted C1-C6 alkoxy" represented by R³ is sometimes referred to as "substituent R^{C}."

The "substituted or unsubstituted C1-C6 alkoxy" represented by R³ is
preferably C1-C6 alkoxy that may have, as the substituent R^{C}, a "4- to 10-membered monocyclic saturated heterocyclic group that may be substituted with at least one kind selected from the group consisting of methyl, ethyl, and amino, and contains 1 to 3 identical or different heteroatoms selected from nitrogen, oxygen, and sulfur," and
more preferably C1-C6 alkoxy that may have, as the substituent R^{C}, a "4- to 10-membered monocyclic saturated heterocyclic group containing 1 to 3 identical or different heteroatoms selected from nitrogen, oxygen, and sulfur."

Examples of the "4- to 10-membered monocyclic saturated heterocyclic group containing 1 to 3 identical or different heteroatoms selected from nitrogen, oxygen, and sulfur" mentioned above as an example of the substituent R^{C} include preferably a "4-to 6-membered monocyclic saturated heterocyclic group containing 1 to 3 identical or different heteroatoms selected from nitrogen, oxygen, and sulfur, " more preferably a "4- to 6-membered monocyclic saturated heterocyclic group containing one oxygen atom," and even more preferably a "5-membered monocyclic saturated heterocyclic group containing one oxygen atom."

The "substituted or unsubstituted C1-C6 alkoxy" represented by R³ is preferably C1-C6 alkoxy that may have, as the substituent R^{C}, a "4- to 10-membered monocyclic saturated heterocyclic group containing 1 to 3 identical or different heteroatoms selected from nitrogen, oxygen, and sulfur,"
more preferably C1-C4 alkoxy that may have, as the substituent R^{C}, a "4- to 6-membered monocyclic saturated heterocyclic group containing 1 to 3 identical or different heteroatoms selected from nitrogen, oxygen, and sulfur,"
even more preferably C1-C4 alkoxy that may have, as the substituent R^{C}, a "4- to 6-memberedmonocyclic saturated heterocyclic group containing one oxygen atom,"
still more preferably methoxy, ethoxy, tetrahydrofuranylmethoxy, tetrahydropyranylmethoxy, tetrahydrofuranylethoxy, or tetrahydropyranylethoxy, and
further still more preferably ethoxy or tetrahydrofuranylmethoxy.

The number of substituent is not limited, but is preferably 0 to 3, and more preferably 0 to 2.

Examples of the "substituent" when each group represented by R³ has a substituent include those mentioned above, and the number thereof is typical one, two, or three.

Examples of the "C1-C6 alkoxy" in the "substituted or unsubstituted C1-C6 alkoxy" represented by R³ include those mentioned above;
preferably C1-C4 alkoxy; and
more preferably methoxy or ethoxy.

In one embodiment, the compound represented by Formula (I) or a salt thereof is as follows:
R¹ is C1-C6 alkoxyalkyl,
R² is C3-C5 cycloalkyl that may have the substituent R^{A}, and
R³ is
   hydrogen,
C2-C6 alkynyl that may have the substituent R^{B}, or
C1-C6 alkoxy that may have the substituent R^{C}.

In one embodiment, the compound represented by Formula (I) or a salt thereof is as follows:
R¹ is C1-C3 alkoxyalkyl,
R² is C3 cycloalkyl that may have the substituent R^{A}, and
R³ is
   C2-C3 alkynyl that may have the substituent R^{B}, or
C1-C2 alkoxy that may have the substituent R^{C}.

In one embodiment, the compound represented by Formula (I) or a salt thereof is as follows:
R¹ is methoxymethyl,
R² is methylcyclopropyl, and
R³ is hydrogen,
propynyl that may be substituted with morpholino, dimethylamino, piperidinyl, pyrrolidinyl, branched propanol, or 3-thiomorpholinyl,
ethynyl that may be substituted with 1,3-dimethylpyrazolyl, 1-methylpiperidinyl, 1-methylpyrazolyl, 1-methylimidazolyl, pyridinyl, or 6-aminopyridinyl,
ethoxy, or
tetrahydrofuranylmethoxy.

In one embodiment, the compound represented by Formula (I) or a salt thereof is as follows:
R¹ is methoxymethyl,
R² is methylcyclopropyl, and
R³ is propynyl that may be substituted with morpholino, dimethylamino, piperidinyl, pyrrolidinyl, branched propanol, 3-thiomorpholinyl, or tetrahydropyranyl,
ethynyl that may be substituted with 1,3-dimethylpyrazolyl, 1-methylpiperidinyl, 1-methylpyrazolyl, 1-methylimidazolyl, pyridinyl, 6-aminopyridinyl, or tetrahydropyranyl,
ethoxy, or
tetrahydrofuranylmethoxy.

In one embodiment, the compound represented by Formula (I) or a salt thereof is as follows:
R¹ is methoxymethyl,
R² is methylcyclopropyl, and
R³ is propynyl that may be substituted with morpholino, dimethylamino, or pyrrolidinyl,
ethynyl that may be substituted with 1,3-dimethylpyrazolyl, 1-methylpyrazolyl, pyridinyl, or tetrahydropyranyl, or ethoxy.

In one embodiment, the compound represented by Formula (I) or a salt thereof is any of the following or a salt thereof:
(1) 4-amino-6-[2-(1,3-dimethyl-1H-pyrazol-4-yl)ethynyl] -N-[4-(methoxymethyl)phenyl]-7-(1-methylcyclopropyl)-7H-pyrrol o[2,3-d]pyrimidine-5-carboxamide;
(2) 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-car boxamide;
(3) 4-amino-N-[4-(methoxymethyl)phenyl]-7-(1-methylcyclopropyl)-6-[(1-methylpiperidin-4-yl)ethynyl]-7H-pyrrolo[2,3-d]pyrimidine-5-carboxamide;
(4) 4-amino-N-[4-(methoxymethyl)phenyl]-6-((1-methyl-1H-pyrazol-4-yl)ethynyl)-7-(1-methylcyclopropyl)-7H-pyrrolo[2,3-d]pyrimidin e-5-carboxamide;
(5) 4-amino-N-[4-(methoxymethyl)phenyl]-7-(1-methylcyclopropyl)-6-(prop-1-yn-1-yl)-7H-pyrrolo[2,3-d]pyrimidine-5-carboxamide;
(6) 4-amino-6-[3-(dimethylamino)prop-1-yn-1-yl]-N-[4-(methoxymethyl)phenyl]-7-(1-methylcyclopropyl)-7H-pyrrolo[2 ,3-d]pyrimidine-5-carboxamide;
(7) (R)-4-amino-N-[4-(methoxymethyl)phenyl]-7-(1-methylcyclopropyl )-6-((tetrahydrofuran-2-yl)methoxy)-7H-pyrrolo[2,3-d]pyrimidin e-5-carboxamide;
(8) 4-amino-N-[4-(methoxymethyl)phenyl]-7-(1-methylcyclopropyl)-7H -pyrrolo[2,3-d]pyrimidine-5-carboxamide;
(9) 4-amino-6-ethoxy-N-[4-(methoxymethyl)phenyl]-7-(1-methylcyclop ropyl)-7H-pyrrolo[2,3-d]pyrimidine-5-carboxamide;
(10) 4-amino-N-(4-(methoxymethyl)phenyl)-6-((1-methyl-1H-imidazol-5 -yl)ethynyl)-7-(1-methylcyclopropyl)-7H-pyrrolo[2,3-d]pyrimidi ne-5-carboxamide;
(11) 4-amino-N-[4-(methoxymethyl)phenyl]-7-(1-methylcyclopropyl)-6-(3-(piperidin-1-yl)prop-1-yn-1-yl)-7H-pyrrolo[2,3-d]pyrimidine -5-carboxamide;
(12) 4-amino-N-[4-(methoxymethyl)phenyl]-7-(1-methylcyclopropyl)-6-(3-(pyrrolidin-1-yl)prop-1-yn-1-yl)-7H-pyrrolo[2,3-d]pyrimidin e-5-carboxamide;
(13) 4-amino-N-[4-(methoxymethyl)phenyl]-7-(1-methylcyclopropyl)-6-((tetrahydro-2H-pyran-4-yl)ethynyl)-7H-pyrrolo[2,3-d]pyrimidin e-5-carboxamide;
(14) 4-amino-6-(4-hydroxy-4-methylpent-1-yn-1-yl)-N-[4-(methoxymeth yl)phenyl]-7-(1-methylcyclopropyl)-7H-pyrrolo[2,3-d]pyrimidine -5-carboxamide;
(15) 4-amino-N-[4-(methoxymethyl)phenyl]-7-(1-methylcyclopropyl)-6-[3-(tetrahydro-2H-pyran-4-yl)prop-1-yn-1-yl]-7H-pyrrolo[2,3-d] pyrimidine-5-carboxamide;
(16) 4-amino-N-[4-(methoxymethyl)phenyl]-7-(1-methylcyclopropyl)-6-(pyridin-3-ylethynyl)-7H-pyrrolo[2,3-d]pyrimidine-5-carboxamid e;
(17) 4-amino-6-[(6-aminopyridin-3-yl)ethynyl]-N-[4-(methoxymethyl)phenyl]-7-(1-methylcyclopropyl)-7H-pyrrolo[2 ,3-d]pyrimidine-5-carboxamide; and
(18) 4-amino-N-[4-(methoxymethyl)phenyl]-7-(1-methylcyclopropyl)-6-(3-thiomorpholinoprop-1-yn-1-yl)-7H-pyrrolo[2,3-d]pyrimidine-5 -carboxamide.

In one embodiment, the compound represented by Formula (I) or a salt thereof is any of the following or a salt thereof:
(1) 4-amino-6-[2-(1,3-dimethyl-1H-pyrazol-4-yl)ethynyl] -N-[4-(methoxymethyl)phenyl]-7-(1-methylcyclopropyl)-7H-pyrrol o[2,3-d]pyrimidine-5-carboxamide;
(2) 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-car boxamide;
(4) 4-amino-N-[4-(methoxymethyl)phenyl]-6-((1-methyl-1H-pyrazol-4-yl)ethynyl)-7-(1-methylcyclopropyl)-7H-pyrrolo[2,3-d]pyrimidin e-5-carboxamide;
(5) 4-amino-N-[4-(methoxymethyl)phenyl]-7-(1-methylcyclopropyl)-6-(prop-1-yn-1-yl)-7H-pyrrolo[2,3-d]pyrimidine-5-carboxamide;
(6) 4-amino-6-[3-(dimethylamino)prop-1-yn-1-yl]-N-[4-(methoxymethyl)phenyl]-7-(1-methylcyclopropyl)-7H-pyrrolo[2 ,3-d]pyrimidine-5-carboxamide;
(9) 4-amino-6-ethoxy-N-[4-(methoxymethyl)phenyl]-7-(1-methylcyclop ropyl)-7H-pyrrolo[2,3-d]pyrimidine-5-carboxamide;
(12) 4-amino-N-[4-(methoxymethyl)phenyl]-7-(1-methylcyclopropyl)-6-(3-(pyrrolidin-1-yl)prop-1-yn-1-yl)-7H-pyrrolo[2,3-d]pyrimidin e-5-carboxamide;
(13) 4-amino-N-[4-(methoxymethyl)phenyl]-7-(1-methylcyclopropyl)-6-((tetrahydro-2H-pyran-4-yl)ethynyl)-7H-pyrrolo[2,3-d]pyrimidin e-5-carboxamide; and
(16) 4-amino-N-[4-(methoxymethyl)phenyl]-7-(1-methylcyclopropyl)-6-(pyridin-3-ylethynyl)-7H-pyrrolo[2,3-d]pyrimidine-5-carboxamid e.

The present invention also provides : a RET inhibitor comprising the compound (I) or a salt thereof (e.g., the compound or a salt thereof described as the active ingredient of the brain-penetrable antitumor agent according to any one of [1] to [9] above. The same applies hereinafter) as an active ingredient; a method for treating a subject having a tumor, comprising administering a brain-penetrable antitumor agent comprising an effective amount of the compound (I) or a salt thereof to the subject in need of treatment; a commercial package comprising the compound (I) or a salt thereof serving as an active ingredient, and an instruction manual for use thereof for treating a tumor in a subject through use of a brain-penetrable antitumor agent; a brain-penetrable antitumor agent for treating a tumor with enhanced activation of RET, comprising the compound (I) or a salt thereof as an active ingredient; a method for treating a subject having a tumor with enhanced activation of RET, comprising administering a brain-penetrable antitumor agent comprising an effective amount of the compound (I) or a salt thereof to the subject; a commercial package comprising the compound or a salt thereof represented by the compound (I) or a salt thereof serving as an active ingredient, and an instruction manual for use thereof for treating a tumor with enhanced activation of RET in a subject through use of a brain-penetrable antitumor agent; and the like.

The compound (I) and a salt thereof may be produced by a known organic synthesis method. For example, the compound (I) and a salt thereof may be produced by a method described in International Publication No. WO2017/146116 or the like.

When the compound (I) has isomers, such as optical isomers, stereoisomers, rotational isomers, and tautomers, mixtures of any of the isomers are included within the scope of the compound (I), unless otherwise specified. For example, when the compound (I) has optical isomers, the optical isomer separated from a racemic mixture is also included within the scope of the compound (I) , unless otherwise specified.

The salt of the compound (I) refers to a pharmaceutically acceptable salt. Examples of the salt include base addition salts and acid addition salts.

The term "pharmaceutically acceptable salt" refers to a salt having desired pharmacological activity of the compound, the salt being prepared from any of pharmaceutically acceptable nontoxic bases or acids including inorganic or organic bases and inorganic or organic acids.

Examples of such salt specifically include acid addition salts with inorganic acids, such as hydrochloric acid, hydrobromic acid, hydroiodic acid, sulfuric acid, nitric acid, and phosphoric acid; acid addition salts with organic acids, such as formic acid, acetic acid, propionic acid, oxalic acid, malonic acid, succinic acid, fumaric acid, maleic acid, lactic acid, malic acid, citric acid, tartaric acid, carbonic acid, picric acid, methanesulfonic acid, p-toluenesulfonic acid, and glutamic acid; salts with inorganic bases, such as sodium, potassium, magnesium, calcium, and aluminum; salts with organic bases, such as methylamine, ethylamine, meglumine, and ethanolamine; salts with basic amino acids, such as lysine, arginine, and ornithine; and ammonium salts.

The compound (I) or a salt thereof includes a prodrug thereof as well. The prodrug refers to a compound that can be converted to the compound (I) or a salt thereof through a reaction with an enzyme, gastric acid, or the like, under physiological conditions in vivo, i.e., a compound that can be converted to the compound (I) or a salt thereof by enzymatic oxidation, reduction, hydrolysis, or the like; or a compound that can be converted to the compound (I) or a salt thereof by hydrolysis with gastric acid or the like. Further, the prodrug may be a compound that can be converted to the compound (I) or a salt thereof under physiological conditions, such as those described in *"*Iyakuhin no Kaihatsu [Development of Pharmaceuticals]," Vol. 7, Molecular Design, published in 1990 by Hirokawa Shoten Co., pp. 163-198.

The compound (I) or a salt thereof may be amorphous or crystalline. Single crystals and polymorphic mixtures are included within the scope of the compound (I) or a salt thereof. Such crystals can be produced by crystallization according to a known crystallization method. The compound (I) or a salt thereof may be a solvate (e.g., a hydrate) or a non-solvate. Any of such forms are included within the scope of the compound (I) or a salt thereof. A compound labeled with an isotope (e.g., ³H, ¹⁴C, ³⁵S, ¹²⁵I, etc.) or the like is also included within the scope of the compound (I) or a salt thereof. A plurality of crystals (crystalline polymorphs) different from each other in spatially regular atomic arrangement and physicochemical properties may be produced, and the salt according to the present invention may be any of those crystalline polymorphs, or may be a mixture of two or more crystalline polymorphs, or even a mixture of crystalline and amorphous substances.

In the present specification, the term "effective amount" of the compound (I) refers to an amount (therapeutically effective amount) in which the compound (I), for example, induces a biological or medical response of a subject, such as reduction or inhibition of enzymatic or protein activity; ameliorates a symptom; relieves a condition; slows or delays the progression of a disease; or prevents a disease.

In the present specification, "treatment" includes postoperative adjuvant chemotherapy to be performed for the prevention of recurrence after surgical removal of a tumor, and preoperative adjuvant chemotherapy to be performed in advance to surgically remove a tumor.

In the present specification, the term "subject" includes mammals and non-mammals. Examples of the mammals include, but are not limited to, humans, chimpanzees, apes, monkeys, cattle, horses, sheep, goats, pigs, rabbits, dogs, cats, rats, mice, guinea pigs, etc. Examples of the non-mammals include, but are not limited to, birds, fishes, reptiles, etc. In one embodiment, the subject is a mammal, in particular, a human, and may be a human diagnosed to need treatment for a symptom, a condition, or a disease disclosed in the present specification.

The age of the subject to which the therapeutic agent of the present invention is administered is not particularly limited. The therapeutic agent of the present invention may be used for not only adults, but also elderly people or children.

The compound (I) or a salt thereof, having an excellent RET inhibitory activity, is useful as a pharmaceutical preparation for preventing and/or treating RET-related diseases. Examples of the "RET-related diseases" include diseases whose incidence can be reduced, and whose symptoms can be remitted, relieved, and/or completely cured by eliminating, suppressing, and/or inhibiting RET function. Examples of such diseases include, but are not limited to, malignant tumors, etc. The malignant tumor is preferably a malignant tumor with enhanced activation of RET; more preferably salivary gland cancer, lung cancer (non-small cell lung cancer, small cell lung cancer, mesothelioma, etc.), colorectal cancer (colon cancer, rectal cancer, etc.), thyroid cancer, breast cancer, pancreas cancer, leukemia, skin cancer, malignant melanoma, or brain tumor with enhanced activation of RET; even more preferably salivary gland cancer, lung cancer, breast cancer, pancreas cancer, colorectal cancer, ovarian cancer, thyroid cancer, skin cancer, malignant melanoma, or brain tumor; and particularly preferably non-small cell lung cancer, breast cancer, colorectal cancer, thyroid cancer, or brain tumor.

The phrase "enhanced activation of RET" indicates that the activated state of RET is enhanced due to, for example, translocations and mutations (including point mutations, deletion mutations, and insertion mutations) of the RET gene, and overexpression (including an increased copy numbers of the RET gene, overexpression of messenger RNA of RET, increased RET proteins, and constitutively activated RET proteins).

The type of cancer and tumor to be treated by the compound or a salt thereof of the present invention is not particularly limited. Examples include epithelial cancers (respiratory organ cancers, digestive organ cancers, reproductive organ cancers, secretion organ cancers, etc.), sarcomas, hematopoietic tumors, central nervous system tumors, and peripheral nerve tumors.

Specific examples of the type of cancer include head and neck cancer, thyroid cancer, salivary gland cancer, esophagus cancer, gastric cancer (digestive organ cancers), duodenal cancer, liver cancer, biliary tract cancer (gallbladder, cholangiocarcinoma, etc.), pancreas cancer, colorectal cancer (colon cancer, rectal cancer, etc.), lung cancer (non-small cell lung cancer, small cell lung cancer, mesothelioma, etc.), breast cancer, ovarian cancer, uterine cancer (cervical cancer, endometrial cancer, etc.), renal

cancer, renal pelvis-ureteral cancer, bladder cancer, prostate cancer, testicular tumor, leukemia, malignant lymphoma, multiple myeloma, osteosarcoma, soft-tissue sarcoma, skin cancer, malignant melanoma, adrenal tumor, brain tumor, and the like. The target cancer is preferably salivary gland cancer, lung cancer (non-small cell lung cancer, small cell lung cancer, mesothelioma, etc.), colorectal cancer (colon cancer, rectal cancer, etc.), thyroid cancer, breast cancer, leukemia, skin cancer, malignant melanoma, or brain tumor; more preferably salivary gland cancer, lung cancer (non-small cell lung cancer, small cell lung cancer, mesothelioma, etc.), colorectal cancer (colon cancer, rectal cancer, etc.), thyroid cancer, breast cancer, pancreas cancer, leukemia, skin cancer, malignant melanoma, or brain tumor; even more preferably salivary gland cancer, lung cancer, breast cancer, pancreas cancer, colorectal cancer, ovarian cancer, thyroid cancer, skin cancer, malignant melanoma, or brain tumor; and particularly preferably non-small cell lung cancer, breast cancer, colorectal cancer, thyroid cancer, or brain tumor.

In a typical embodiment of the present invention, the tumor on which the compound (I) exhibits an effect by passing through the BBB is a brain tumor. In the present invention, the brain tumor includes a primary brain tumor and a metastatic brain tumor . Examples of the primary tumor include, but are not particularly limited to, glioma, primary central nervous system lymphoma, meningioma, pituitary adenoma, schwannoma, craniopharyngioma, etc. Moreover, the type of tumor serving as a primary site for the metastatic brain tumor is not limited.

In the case of the metastatic brain tumor, the type of tumor serving as a primary site therefor is not limited. Specific examples of the type of cancer include head and neck cancer, thyroid cancer, salivary gland cancer, esophagus cancer, gastric cancer (digestive organ cancers), duodenal cancer, liver cancer, biliary tract cancer (gallbladder, cholangiocarcinoma, etc.), pancreas cancer, colorectal cancer (colon cancer, rectal cancer, etc.), lung cancer (non-small cell lung cancer, small cell lung cancer, mesothelioma, etc.), breast cancer, ovarian cancer, uterine cancer (cervical cancer, endometrial cancer, etc.), renal cancer, renal pelvis-ureteral cancer, bladder cancer, prostate cancer, testicular tumor, leukemia, malignant lymphoma, multiple myeloma, osteosarcoma, soft-tissue sarcoma, skin cancer, malignant melanoma, adrenal tumor, and the like. The target cancer is preferably salivary gland cancer, lung cancer (non-small cell lung cancer, small cell lung cancer, mesothelioma, etc.), colorectal cancer (colon cancer, rectal cancer, etc.), thyroid cancer, breast cancer, pancreas cancer, leukemia, skin cancer, malignant melanoma, or brain tumor; even more preferably salivary gland cancer, lung cancer, breast cancer, pancreas cancer, colorectal cancer, ovarian cancer, thyroid cancer, skin cancer, malignant melanoma, or brain tumor; and particularly preferably non-small cell lung cancer, breast cancer, colorectal cancer, thyroid cancer, or brain tumor.

The active ingredient may be provided alone as the therapeutic agent of the present invention. Further, the therapeutic agent of the present invention may have blended therein, in addition to the compound (I) or a salt thereof serving as the active ingredient, a pharmaceutically acceptable carrier and the like as necessary. Thus, the therapeutic agent of the present invention may be prepared as a pharmaceutical composition comprising one component or two or more components. One mode of the present invention provides a pharmaceutical composition comprising the compound (I) or a salt thereof. The pharmaceutical composition according to one embodiment of the present invention comprises the compound (I) or a salt thereof, and a pharmaceutically acceptable carrier. Moreover, one embodiment of the present invention provides use of the compound (I) or a salt thereof for producing a therapeutic agent or a pharmaceutical composition. Another embodiment of the present invention provides the compound (I) or a salt thereof for use as a therapeutic agent or a pharmaceutical preparation.

The therapeutic agent of the present invention may be produced as any of various preparations to be administered, by a known method using a pharmaceutically acceptable carrier as necessary. The dosage form thereof may be any of oral or parenteral forms. The forms of such preparations are not particularly limited, and examples thereof include: oral agents, such as a tablet, a coated tablet, a pill, a powder, a granule, a capsule, a solution, a suspension, and an emulsions; parenteral agents, such as an injection, a suppository, and an inhalant; etc.

When the formation is performed in the formof a tablet, examples of the pharmaceutically acceptable carrier that may be used include excipients, such as lactose, sucrose, sodium chloride, glucose, urea, starch, calcium carbonate, kaolin, crystalline cellulose, and silicic acid; binders, such as water, ethanol, propanol, corn starch, simple syrup, glucose liquid, starch liquid, gelatin solution, carboxymethyl cellulose, shellac, methyl cellulose, hydroxypropyl cellulose, hydroxypropylmethyl cellulose, potassium phosphate, and polyvinylpyrrolidone; disintegrants, such as dried starch, sodium alginate, agar powder, laminaran powder, sodium hydrogen carbonate, calcium carbonate, polyoxyethylene sorbitan fatty acid esters, sodium lauryl sulfate, stearic acid monoglyceride, and lactose; disintegration-suppressing agents, such as sucrose, stearic acid, cacao butter, and hydrogenated oil; absorption enhancers, such as a quaternary ammonium salt and sodium lauryl sulfate; moisturizing agents, such as glycerin and starch; adsorbents, such as starch, lactose, kaolin, bentonite, and colloidal silicic acid; and lubricants, such as purified talc, a stearic acid salt, boric acid powder, and polyethylene glycol. Further, the tablet may be formed into a tablet subjected to typical coating, such as a sugar-coated tablet, a gelatin-encapsulated tablet, an enteric-coated tablet, a film-coated tablet, a double tablet, or a multi-layered tablet as required.

When the formation is performed in the form of a pill, examples of the pharmaceutically acceptable carrier that may be used include excipients, such as glucose, lactose, starch, cacao butter, hydrogenated vegetable oil, kaolin, and talc; binders, such as gum arabic powder, tragacanth powder, gelatin, and ethanol; and disintegrants, such as laminaran and agar. The capsule is prepared by mixing the compound or a salt thereof with any of the various pharmaceutically acceptable carriers given above as examples and filling the mixture into a hard gelatin capsule, a soft capsule, or the like in accordance with a conventional method.

When the oral liquid preparation is formed, an oral solution, a syrup, an elixir, etc. can be produced by using, for example, a sweetener, a buffer, a stabilizer, etc. as the pharmaceutically acceptable carrier in accordance with a conventional method. In this case, examples include sweeteners, such as sucrose, orange peel, citric acid, and tartaric acid; buffers, such as sodium citrate; and stabilizers, such as tragacanth, gum arabic, and gelatin.

When the formation is performed in the form of a suppository, examples of the pharmaceutically acceptable carrier that may be used include polyethylene glycol, cacao butter, a higher alcohol, a higher alcohol ester, gelatin, semisynthetic glyceride and the like.

When the injection is formed, it is preferred that the solution, the emulsion, and the suspension be sterilized, and be isotonic to blood. In addition, in the formation into those forms, a diluent may be used as the pharmaceutically acceptable carrier, and examples of such diluent that may be used include water, a lactic acid aqueous solution, ethyl alcohol, propylene glycol, macrogol, ethoxylated isostearyl alcohol, polyoxyethylenated isostearyl alcohol, and polyoxyethylene sorbitan fatty acid esters.

In this case, sodium chloride, glucose, or glycerol in an amount sufficient for preparing an isotonic solution may be incorporated into pharmaceutical preparations, and a general solubilizing agent, buffer, soothing agent, etc. may be added.

In the case of an inhalant, examples thereof include various forms, such as an aerosol, a powder inhalant, and a liquid inhalant.

Further, each of such preparations may be blended with a colorant, a preservative, a perfume, a flavoring agent, a sweetening agent, or the like as a pharmaceutically acceptable carrier as necessary, and/or with other pharmaceuticals.

In the present invention, the term "daily dose" refers to the amount of the active ingredient to be administered per day. In the therapeutic agent of the present invention, the daily dose on days on which the compound (I) or a salt thereof is administered depends on the condition, body weight, age, gender, etc., of the patient, and cannot be generalized. For example, the dose of the compound (I) for an adult (body weight: 50 kg) is generally preferably 10 to 2000 mg/day, more preferably 20 to 1500 mg/day, and even more preferably 40 to 1200 mg/day.

Moreover, the amount of the compound (1) to be incorporated in each of such dosage unit forms depends on the condition of the patient to whom the compound is administered, the dosage form, etc. In general, in the case of an oral agent, an injection, and a suppository, the amount of the compound is preferably 0.05 to 1000 mg, 0.01 to 500 mg, and 1 to 1000 mg, respectively, per dosage unit form.

The method for administering the therapeutic agent of the present invention is appropriately determined depending on various forms of preparations, the age, gender, and other conditions of the patient, the degrees of symptoms of the patient, etc. The method for administering the therapeutic agent of the present invention is not limited; however, since its active ingredient, the compound (I) or a salt thereof, shows an excellent brain penetration property, it is preferable to use an administration method that allows penetration to the brain via the systemic circulatory blood flow. Examples of the method for administering the therapeutic agent of the present invention include oral administration, intravenous administration, intraarterial administration, intramuscular administration, intradermal administration, subcutaneous administration, intraperitoneal administration, intrarectal administration, and the like. In the present invention, at least two of these administration methods may be combined. For example, a tablet, a pill, a powder, a granule, a capsule, a solution, a suspension, and an emulsion are orally administered. An injection is intravenously administered alone or as a mixture with a general adjuvant, such as glucose or an amino acid, or as necessary, administered alone intraarterially, intramuscularly, intradermally, subcutaneously, or intraperitoneally.A suppository is intrarectally administered.

In the present invention, the "instruction manual" refers to, without limitation, the accompanying document provided with the medicament, including an instruction manual that is displayed by electronic means, such as a bar code or two-dimensional code to access the instruction manual on the box used to store the medicament.

The following describes the present invention in more detail with reference to Examples. However, the present invention is not limited to the Examples.

All documents and publications mentioned in the present specification are incorporated herein by reference in their entirety irrespective of their purposes.

Moreover, the purposes, features, and advantages of the present invention and ideas thereof are apparent to persons skilled in the art from the description of the present specification, and persons skilled in the art could easily carry out the present invention on the basis of the description of the present specification. The best mode for carrying out the invention, specific Examples, and the like are illustrative of preferred embodiments of the present invention and are shown for illustration or description, and the present invention is not limited thereto. It is apparent to persons skilled in the art that various modifications are possible on the basis of the description of the present specification within the spirit and scope of the present invention disclosed in the present specification.

### [Examples]

In the following Examples, % represents mass percent, unless otherwise specified.

Commercially available reagents were used in the Examples, unless otherwise specified.

The following are the abbreviations used and the meaning of each.
PBS: phosphate buffered saline
LC-MS/MS: liquid chromatograph-tandem mass spectrometer
HPMC: hydroxypropyl methylcellulose

### Test Example 1: Evaluation of Plasma Protein Binding and Brain Protein Binding

Compounds shown in Tables 1 to 4 were added to mouse plasma at a final concentration of 1 µmol/L. The resultant compound-added mouse plasma was added to the donor side of an equilibrium dialysis device (HTDialysis) in which an equilibrium dialysis membrane was set. An equal amount of PBS was added to the receiver side of the device, and after incubation in a 10% CO2 incubator for 6 hours, the compounds on the donor side and the receiver side were detected with the LC-MS/MS. The unbound ratio of each compound to plasma protein was calculated from the ratio of the compound peak area of the receiver side to that of the donor side.

The unbound ratio of each compound to brain protein was calculated in the same manner as above except that: the compound was added to a mouse brain homogenate instead of the mouse plasma; and a dilution ratio was taken into consideration in the calculation of the unbound ratio. In this case, the mouse brain homogenate was obtained by adding a 3-fold amount of PBS to the mouse brain, and then homogenizing the mixture through use of an ultrasonic homogenizer.

### Test Example 2: Evaluation of Brain Penetration Property

The compounds shown in Tables 1 to 4 were dissolved or suspended in 0.5% HPMC, 0.1N hydrochloric acid, and orally administered in a single dose to BALB/cAJcl-nu/nu mice (CLEA Japan, Inc.) untreated, or subcutaneously implanted with TT cells, or subcutaneously implanted with the BaF3/KIF5B-RET_RFP cell line. After 1 hour from the oral administration, blood was collected from the inferior vena cava under isoflurane anesthesia, and then the whole brain was taken out. Thus, blood and brain samples were obtained. The obtained blood sample was centrifuged to obtain a plasma sample. A 3-fold amount of water was added to the obtained brain sample, and then the mixture was homogenized through use of an ultrasonic homogenizer to obtain a brain homogenate.

Compound concentrations in the obtained plasma and in the brain homogenate were measured with the LC-MS/MS, and the compound concentration in the brain homogenate was multiplied by a factor of 4 to calculate a compound concentration in the brain. A Kp value was calculated from the ratio of the compound concentrations in the brain/plasma. Unbound compound concentrations in the plasma and in the brain were calculated from the plasma protein unbound ratio and brain protein unbound ratio of each compound determined in Test Example 1, and a Kp,uu value was calculated from the ratio of the unbound compound concentrations in the brain/plasma. A brain penetration property was evaluated on the basis of the calculated Kp value and Kp,uu value (Tables 1 to 4) . Specifically, a compound showing a Kp value of 0.1 or more was judged to have a brain penetration property, and a compound showing a Kp,uu value of 0.3 or more was judged to have an excellent brain penetration property (Varadharaj an, S., et al. (2015) J Pharm Sci 104, 1197-1206). In Table 4, "N/A" in the column "Kp,uu value" means that the value was unmeasured.

The results shown in Tables 1 to 4 revealed that the compound (I) showed a high Kp value and a high Kp,uu value, indicating an excellent brain penetration property.

**[Table 1]**

| Example No. | Structural formula | Kp | Kp,uu |
|---|---|---|---|
| 1 | 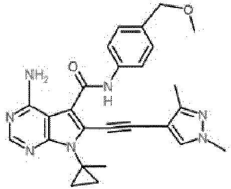 | 0.425 | 0.425 |
| 2 | 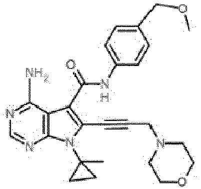 | 1.706 | 1.798 |
| 3 | 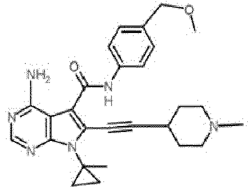 | 0.772 | 0.220 |
| 4 | 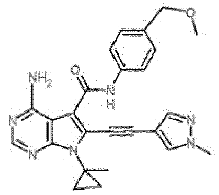 | 1.440 | 1.200 |
| 5 | 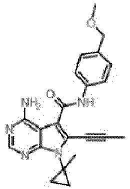 | 1.556 | 0.681 |
| 6 | 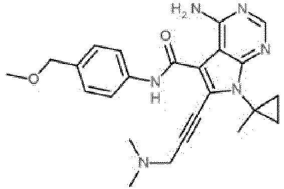 | 0.969 | 0.922 |

**[Table 2]**

| | | | |
|---|---|---|---|
| 7 | 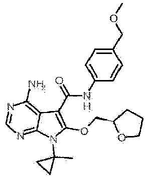 | 1.633 | 0.608 |
| 8 | 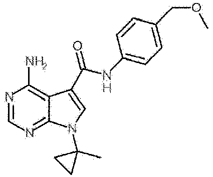 | 0.175 | 0.100 |
| 9 | 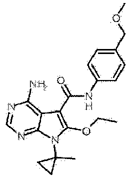 | 2.116 | 0.914 |
| 10 | 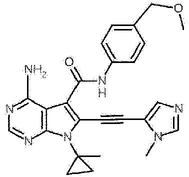 | 0.143 | 0.056 |
| 11 | 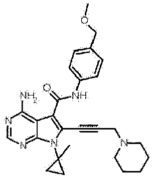 | 1.358 | N/A |
| 12 | 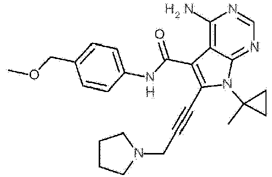 | 1.024 | 1.447 |

**[Table 3]**

| | | | |
|---|---|---|---|
| 13 | 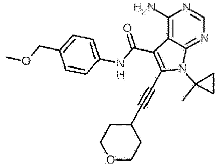 | 2.078 | 0.891 |
| 14 | 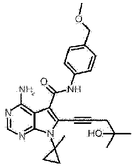 | 0.352 | 0.242 |
| 15 | 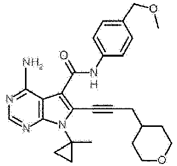 | 0.740 | 0.370 |
| 16 | 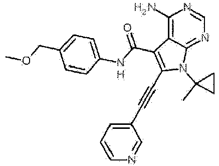 | 1.088 | 0.653 |
| 17 | 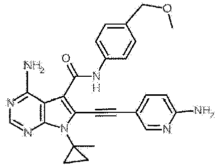 | 0.129 | 0.052 |
| 18 | 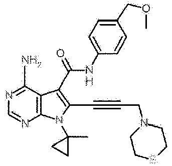 | 0.685 | 0.443 |

**[Table 4]**

| Comparative Example No. | Structural formula | Kp | Kp, uu |
|---|---|---|---|
| 1 | 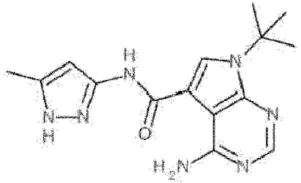 | 0.056 | N/A |
| 2 | 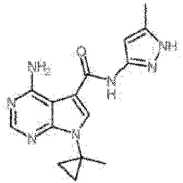 | 0.022 | 0.005 |
| 3 | 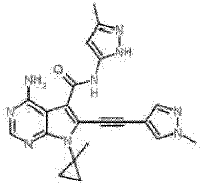 | 0.076 | N/A |

### Test Example 3: Evaluation of Drug Efficacy in Brain Tumor Implantation Model

Antitumor evaluation was performed in models with tumors implanted in the brain. The compounds of Examples 2 and 4 shown in Table 1, and the compound of Example 13 shown in Table 3 were confirmed to have an inhibitory effect on the growth of the implanted tumors.

The growth inhibitory effect was confirmed by using NIH/3T3 cells (NIH/3T3_CCDC6-RET cells) in which luciferase and CCDC6-RET fusion protein were forced to be expressed for gene transfer. Six-week-old male nude mice (BALB/cAJcl-nu/nu, CLEA Japan, Inc.) were fixed on a brain stereotaxic instrument, and 2.5 × 10⁴ cells per mice were implanted at a site 0.5 mm anteriorly from the bregma, 2 to 2.2 mm to the right and 3.5 mm deep. The day of implantation was defined as Day 0, luciferin (150 mg/kg) was administered intraperitoneally on Day 4, and intensity of luminescence (photons/sec) was measured using an IVIS Imaging System (Lumina II, PerkinElmer). According to the measured the intensity of luminescence, the animals were assigned to 4 groups (N=10) so that the average intensity of luminescence of each group was similar (only the control group was evaluated with N=9 because one death due to an accident was observed).

From the next day (Day 5), compounds were administered twice a day for 16 consecutive days. Each compound was suspended in 0.5% HPMC/0.1N hydrochloric acid and administered orally at 50 mg/kg/day. To confirm tumor growth, intensity of luminescence was measured at intervals of 3 or 4 days by the method described above.

The day after the last dose (Day 21) was defined as the last day of measurement, and the inhibitory effect of the compounds on tumor growth was confirmed. However, since a humane endpoint due to tumor growth was applied to the drug non-administration group (control group), statistical evaluation was performed by Dunnett's test using the measurement value on Day 14.

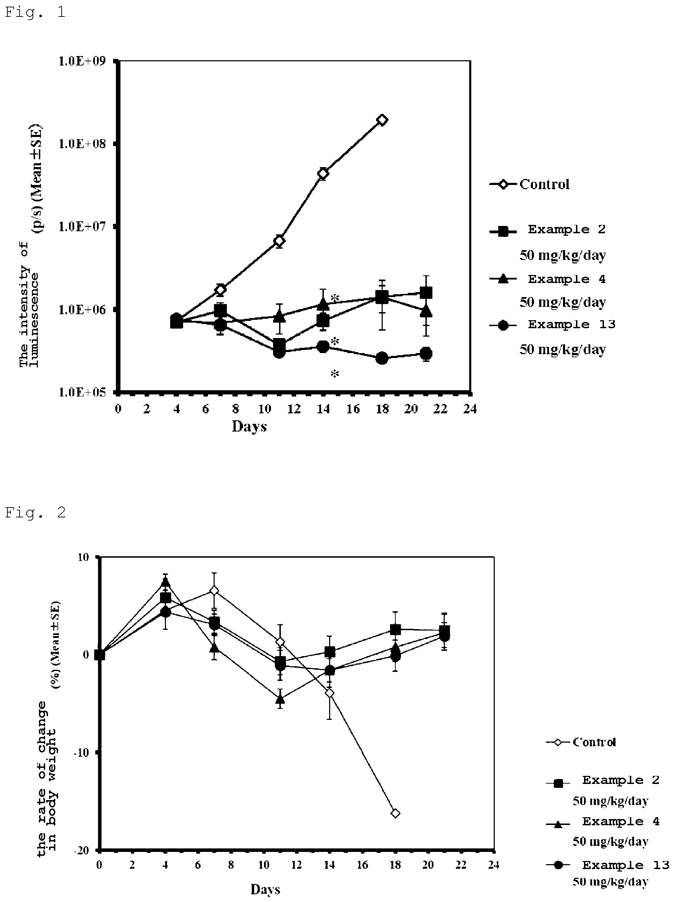

Fig. 1 shows changes in the intensity of luminescence over time, and Fig. 2 shows changes in body weight. In the control group, an increase in the intensity of luminescence was observed, suggesting that the implanted tumors were grown. In addition, weight loss was observed, possibly due to tumor growth. By Day 19, all the animals had lost weight and were deemed unable to continue the test, so a humane endpoint was applied. On the other hand, in the compound-treated groups, there was no significant increase in the intensity of luminescence for all the compounds, suggesting that tumor growth was suppressed. On Day 14, statistical analysis of the intensity of luminescence showed a significant difference (*: p<0.05) in all the compound-treated groups compared to the control group. This suggests that compound administration had a significant inhibitory effect on tumor growth. Furthermore, all animals in the compound-treated groups were able to complete the administration of compounds until Day 22, suggesting that compound administration can be expected to also prolong life.

These results suggest that administration of compounds showing a brain penetration property significantly suppresses the growth of tumor cells implanted in the brain and can be expressed to also prolong life.

## Claims

1. A brain-penetrable antitumor agent comprising a compound represented by Formula (I) below or a salt thereof as an active ingredient: 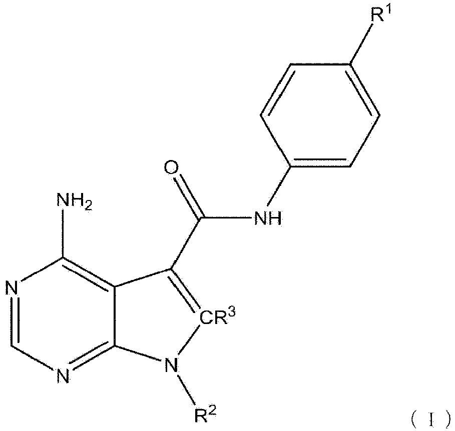 wherein
R¹ is C1-C6 alkoxyalkyl,
R² is substituted or unsubstituted C3-C5 cycloalkyl, and
R³ is hydrogen,
substituted or unsubstituted C2-C6 alkynyl, or
substituted or unsubstituted C1-C6 alkoxy.

2. The brain-penetrable antitumor agent according to claim 1 , wherein the substituted or unsubstituted C3-C5 cycloalkyl represented by R² is substituted with:
(1-1) C1-C2 alkyl.

3. The brain-penetrable antitumor agent according to claim 1 or 2, wherein R² is C3-C4 cycloalkyl that may be substituted with methyl.

4. The brain-penetrable antitumor agent according to any one of claims 1 to 3, wherein the substituted or unsubstituted C2-C6 alkynyl represented by R³ is substituted with:
(2-1) substituted or unsubstituted amino;
(2-2) substituted or unsubstituted C1-C6 alkyl;
(2-3) a substituted or unsubstituted 4- to 10-membered monocyclic saturated heterocyclic group containing 1 to 3 identical or different heteroatoms selected from nitrogen, oxygen, and sulfur; or
(2-4) a substituted or unsubstituted 4- to 10-membered monocyclic unsaturated heterocyclic group containing 1 to 3 identical or different heteroatoms selected from nitrogen, oxygen, and sulfur.

5. The brain-penetrable antitumor agent according to any one of claims 1 to 4, wherein the substituted or unsubstituted C1-C6 alkoxy represented by R³ is substituted with:
(3-1) amino;
(3-2) C1-C6 alkyl that may have hydroxy;
(3-3) a 4- to 10-membered monocyclic saturated heterocyclic group that may be substituted with at least one kind selected from the group consisting of methyl, ethyl, and amino, and contains 1 to 3 identical or different heteroatoms selected from nitrogen, oxygen, and sulfur; or
(3-4) a 4- to 10-membered monocyclic unsaturated heterocyclic group that may be substituted with at least one kind selected from the group consisting of methyl, ethyl, and amino, and contains 1 to 3 identical or different heteroatoms selected from nitrogen, oxygen, and sulfur.

6. The antitumor agent according to any one of claims 1 to 5, wherein
R¹ is C1-C6 alkoxy C1-C6 alkyl,
R² is C3-C5 cycloalkyl that may be substituted with C1-C2 alkyl, and
R³ is
substituted or unsubstituted C2-C6 alkynyl; or
substituted or unsubstituted C1-C6 alkoxy,
wherein the substituted or unsubstituted C2-C6 alkynyl represented by R³ is substituted with:
amino;
C1-C6 alkyl that may be substituted with at least one kind selected from the group consisting of hydroxy, amino, and cyano;
a 4- to 10-membered monocyclic saturated heterocyclic group that may be substituted with at least one kind selected from the group consisting of C1-C6 alkyl, hydroxy, amino, and cyano, and contains 1 to 3 identical or different heteroatoms selected from nitrogen, oxygen, and sulfur; or
a 4- to 10-membered monocyclic unsaturated heterocyclic group that may be substituted with at least one kind selected from the group consisting of C1-C6 alkyl, hydroxy, amino, and cyano, and contains 1 to 3 identical or different heteroatoms selected from nitrogen, oxygen, and sulfur, and
the substituted or unsubstituted C1-C6 alkoxy represented by R³ is substituted with:
amino;
C1-C6 alkyl that may have hydroxy;
a 4- to 10-membered monocyclic saturated heterocyclic group that may be substituted with at least one kind selected from the group consisting of methyl, ethyl, and amino, and contains 1 to 3 identical or different heteroatoms selected from nitrogen, oxygen, and sulfur; or
a 4- to 10-membered monocyclic unsaturated heterocyclic group that may be substituted with at least one kind selected from the group consisting of methyl, ethyl, and amino, and contains 1 to 3 identical or different heteroatoms selected from nitrogen, oxygen, and sulfur.

7. The brain-penetrable antitumor agent according to any one of claims 1 to 6, wherein the compound represented by Formula (I) or a salt thereof is any of the following or a salt thereof:
(1) 4-amino-6-[2-(1,3-dimethyl-1H-pyrazol-4-yl)ethynyl] -N-[4-(methoxymethyl)phenyl]-7-(1-methylcyclopropyl)-7H-pyrrol o[2,3-d]pyrimidine-5-carboxamide;
(2) 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-car boxamide;
(3) 4-amino-N-[4-(methoxymethyl)phenyl]-7-(1-methylcyclopropyl)-6-[(1-methylpiperidin-4-yl)ethynyl]-7H-pyrrolo[2,3-d]pyrimidine-5-carboxamide;
(4) 4-amino-N-[4-(methoxymethyl)phenyl]-6-((1-methyl-1H-pyrazol-4-yl)ethynyl)-7-(1-methylcyclopropyl)-7H-pyrrolo[2,3-d]pyrimidin e-5-carboxamide;
(5) 4-amino-N-[4-(methoxymethyl)phenyl]-7-(1-methylcyclopropyl)-6-(prop-1-yn-1-yl)-7H-pyrrolo[2,3-d]pyrimidine-5-carboxamide;
(6) 4-amino-6-[3-(dimethylamino)prop-1-yn-1-yl]-N-[4-(methoxymethyl)phenyl]-7-(1-methylcyclopropyl)-7H-pyrrolo[2 ,3-d]pyrimidine-5-carboxamide;
(7) (R)-4-amino-N-[4-(methoxymethyl)phenyl]-7-(1-methylcyclopropyl )-6-((tetrahydrofuran-2-yl)methoxy)-7H-pyrrolo[2,3-d]pyrimidin e-5-carboxamide;
(8) 4-amino-N-[4-(methoxymethyl)phenyl]-7-(1-methylcyclopropyl)-7H -pyrrolo[2,3-d]pyrimidine-5-carboxamide;
(9) 4-amino-6-ethoxy-N-[4-(methoxymethyl)phenyl]-7-(1-methylcyclop ropyl)-7H-pyrrolo[2,3-d]pyrimidine-5-carboxamide;
(10) 4-amino-N-(4-(methoxymethyl)phenyl)-6-((1-methyl-1H-imidazol-5 -yl)ethynyl)-7-(1-methylcyclopropyl)-7H-pyrrolo[2,3-d]pyrimidi ne-5-carboxamide;
(11) 4-amino-N-[4-(methoxymethyl)phenyl]-7-(1-methylcyclopropyl)-6-(3-(piperidin-1-yl)prop-1-yn-1-yl)-7H-pyrrolo[2,3-d]pyrimidine -5-carboxamide;
(12) 4-amino-N-[4-(methoxymethyl)phenyl]-7-(1-methylcyclopropyl)-6-(3-(pyrrolidin-1-yl)prop-1-yn-1-yl)-7H-pyrrolo[2,3-d]pyrimidin e-5-carboxamide;
(13) 4-amino-N-[4-(methoxymethyl)phenyl]-7-(1-methylcyclopropyl)-6-((tetrahydro-2H-pyran-4-yl)ethynyl)-7H-pyrrolo[2,3-d]pyrimidin e-5-carboxamide;
(14) 4-amino-6-(4-hydroxy-4-methylpent-1-yn-1-yl)-N-[4-(methoxymeth yl)phenyl]-7-(1-methylcyclopropyl)-7H-pyrrolo[2,3-d]pyrimidine -5-carboxamide;
(15) 4-amino-N-[4-(methoxymethyl)phenyl]-7-(1-methylcyclopropyl)-6-[3-(tetrahydro-2H-pyran-4-yl)prop-1-yn-1-yl]-7H-pyrrolo[2,3-d] pyrimidine-5-carboxamide;
(16) 4-amino-N-[4-(methoxymethyl)phenyl]-7-(1-methylcyclopropyl)-6-(pyridin-3-ylethynyl)-7H-pyrrolo[2,3-d]pyrimidine-5-carboxamid e;
(17) 4-amino-6-[(6-aminopyridin-3-yl)ethynyl]-N-[4-(methoxymethyl)phenyl]-7-(1-methylcyclopropyl)-7H-pyrrolo[2 ,3-d]pyrimidine-5-carboxamide; and
(18) 4-amino-N-[4-(methoxymethyl)phenyl]-7-(1-methylcyclopropyl)-6-(3-thiomorpholinoprop-1-yn-1-yl)-7H-pyrrolo[2,3-d]pyrimidine-5 -carboxamide.

8. The brain-penetrable antitumor agent according to any one of claims 1 to 7, wherein the compound represented by Formula (I) or a salt thereof is any of the following or a salt thereof:
(1) 4-amino-6-[2-(1,3-dimethyl-1H-pyrazol-4-yl)ethynyl] -N-[4-(methoxymethyl)phenyl]-7-(1-methylcyclopropyl)-7H-pyrrol o[2,3-d]pyrimidine-5-carboxamide;
(2) 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-car boxamide;
(4) 4-amino-N-[4-(methoxymethyl)phenyl]-6-((1-methyl-1H-pyrazol-4-yl)ethynyl)-7-(1-methylcyclopropyl)-7H-pyrrolo[2,3-d]pyrimidin e-5-carboxamide;
(5) 4-amino-N-[4-(methoxymethyl)phenyl]-7-(1-methylcyclopropyl)-6-(prop-1-yn-1-yl)-7H-pyrrolo[2,3-d]pyrimidine-5-carboxamide;
(6) 4-amino-6-[3-(dimethylamino)prop-1-yn-1-yl]-N-[4-(methoxymethyl)phenyl]-7-(1-methylcyclopropyl)-7H-pyrrolo[2 ,3-d]pyrimidine-5-carboxamide;
(9) 4-amino-6-ethoxy-N-[4-(methoxymethyl)phenyl]-7-(1-methylcyclop ropyl)-7H-pyrrolo[2,3-d]pyrimidine-5-carboxamide;
(12) 4-amino-N-[4-(methoxymethyl)phenyl]-7-(1-methylcyclopropyl)-6-(3-(pyrrolidin-1-yl)prop-1-yn-1-yl)-7H-pyrrolo[2,3-d]pyrimidin e-5-carboxamide;
(13) 4-amino-N-[4-(methoxymethyl)phenyl]-7-(1-methylcyclopropyl)-6-((tetrahydro-2H-pyran-4-yl)ethynyl)-7H-pyrrolo[2,3-d]pyrimidin e-5-carboxamide; and
(16) 4-amino-N-[4-(methoxymethyl)phenyl]-7-(1-methylcyclopropyl)-6-(pyridin-3-ylethynyl)-7H-pyrrolo[2,3-d]pyrimidine-5-carboxamid e.

9. The brain-penetrable antitumor agent according to any one of claims 1 to 8, for treating a primary or metastatic brain tumor.

10. Use of a compound represented by Formula (I) below or a salt thereof for producing a brain-penetrable antitumor agent: 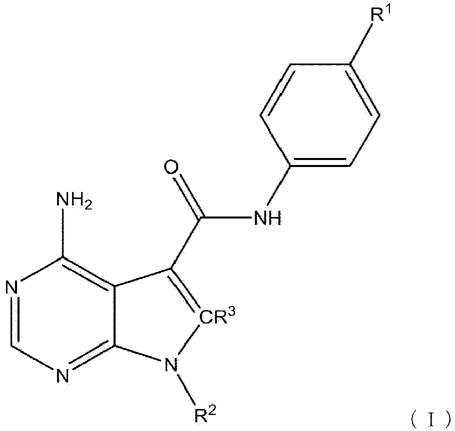 wherein
R¹ is C1-C6 alkoxyalkyl,
R² is substituted or unsubstituted C3-C5 cycloalkyl, and
R³ is hydrogen,
substituted or unsubstituted C2-C6 alkynyl, or
substituted or unsubstituted C1-C6 alkoxy.

11. A method for treating a subject having a tumor, comprising administering a brain-penetrable antitumor agent comprising an effective amount of a compound represented by Formula (I) below or a salt thereof to the subject in need of treatment: 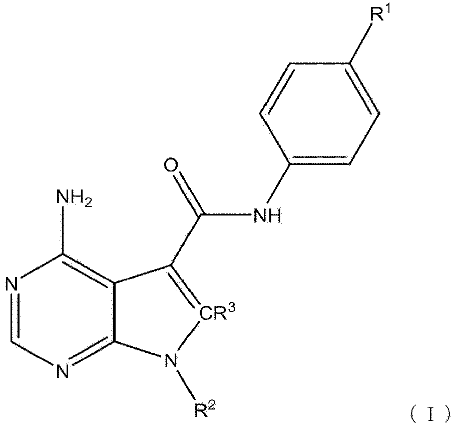 wherein
R¹ is C1-C6 alkoxyalkyl,
R² is substituted or unsubstituted C3-C5 cycloalkyl, and
R³ is hydrogen,
substituted or unsubstituted C2-C6 alkynyl, or
substituted or unsubstituted C1-C6 alkoxy.

12. A compound represented by Formula (I) below or a salt thereof for use in treatment of a tumor through use of a brain-penetrable antitumor agent: 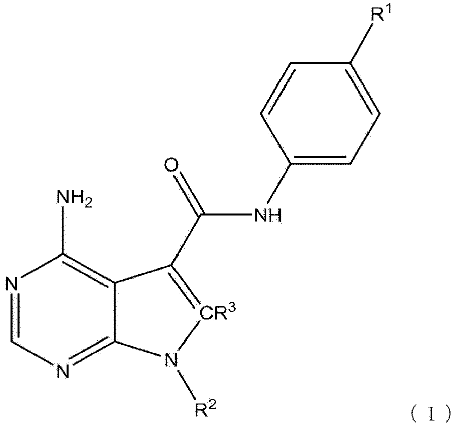 wherein
R¹ is C1-C6 alkoxyalkyl,
R² is substituted or unsubstituted C3-C5 cycloalkyl, and
R³ is hydrogen,
substituted or unsubstituted C2-C6 alkynyl, or
substituted or unsubstituted C1-C6 alkoxy.

13. Use of a compound represented by Formula (I) below or a salt thereof for treating a tumor through use of a brain-penetrable antitumor agent: 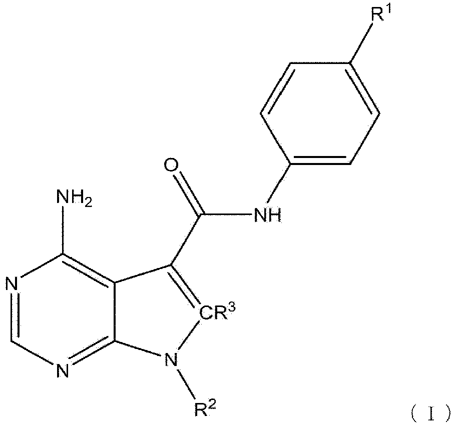 wherein
R¹ is C1-C6 alkoxyalkyl,
R² is substituted or unsubstituted C3-C5 cycloalkyl, and
R³ is hydrogen,
substituted or unsubstituted C2-C6 alkynyl, or
substituted or unsubstituted C1-C6 alkoxy.

14. A commercial package comprising:
a compound represented by Formula (I) below or a salt thereof serving as an active ingredient: 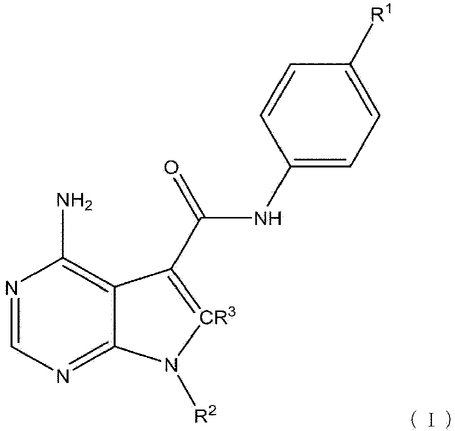 wherein
R¹ is C1-C6 alkoxyalkyl,
R² is substituted or unsubstituted C3-C5 cycloalkyl, and
R³ is hydrogen,
substituted or unsubstituted C2-C6 alkynyl, or
substituted or unsubstituted C1-C6 alkoxy; and
an instruction manual for use thereof for treating a tumor in a subject through use of a brain-penetrable antitumor agent.

15. A brain-penetrable antitumor agent for treating a tumor with enhanced activation of RET, comprising a compound represented by Formula (I) below or a salt thereof as an active ingredient: 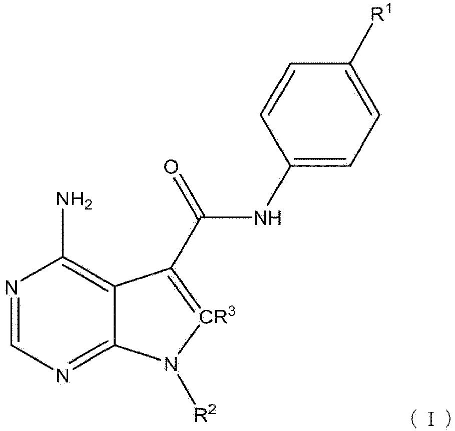 wherein
R¹ is C1-C6 alkoxyalkyl,
R² is substituted or unsubstituted C3-C5 cycloalkyl, and
R³ is hydrogen,
substituted or unsubstituted C2-C6 alkynyl, or
substituted or unsubstituted C1-C6 alkoxy.

16. Use of a compound represented by Formula (I) below or a salt thereof for producing a brain-penetrable antitumor agent for treating a tumor with enhanced activation of RET: 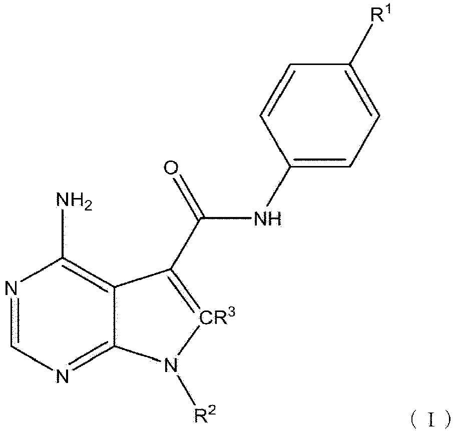 wherein
R¹ is C1-C6 alkoxyalkyl,
R² is substituted or unsubstituted C3-C5 cycloalkyl, and
R³ is hydrogen,
substituted or unsubstituted C2-C6 alkynyl, or
substituted or unsubstituted C1-C6 alkoxy.

17. A method for treating a subject having a tumor with enhanced activation of RET, comprising administering a brain-penetrable antitumor agent comprising an effective amount of a compound represented by Formula (I) below or a salt thereof to the subject: 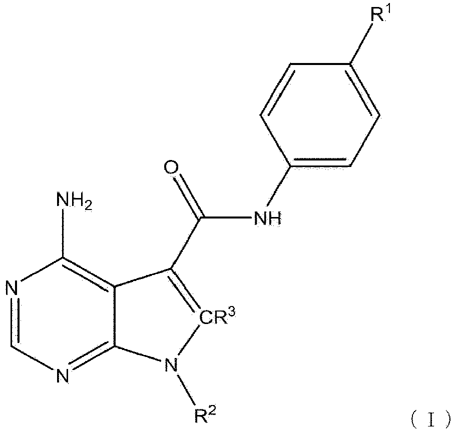 wherein
R¹ is C1-C6 alkoxyalkyl,
R² is substituted or unsubstituted C3-C5 cycloalkyl, and
R³ is hydrogen,
substituted or unsubstituted C2-C6 alkynyl, or
substituted or unsubstituted C1-C6 alkoxy.

18. A compound represented by Formula (I) below or a salt thereof for use in treatment of a tumor with enhanced activation of RET through use of a brain-penetrable antitumor agent: 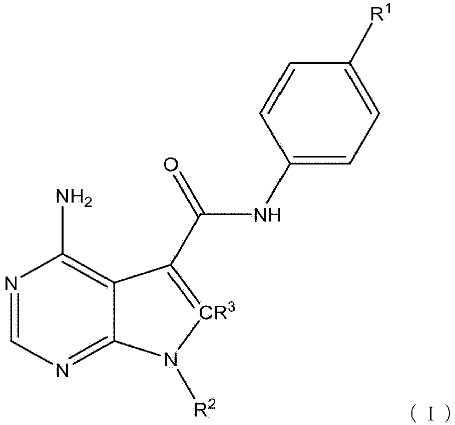 wherein
R¹ is C1-C6 alkoxyalkyl,
R² is substituted or unsubstituted C3-C5 cycloalkyl, and
R³ is hydrogen,
substituted or unsubstituted C2-C6 alkynyl, or
substituted or unsubstituted C1-C6 alkoxy.

19. Use of a compound represented by Formula (I) below or a salt thereof for treating a tumor with enhanced activation of RET through use of a brain-penetrable antitumor agent: 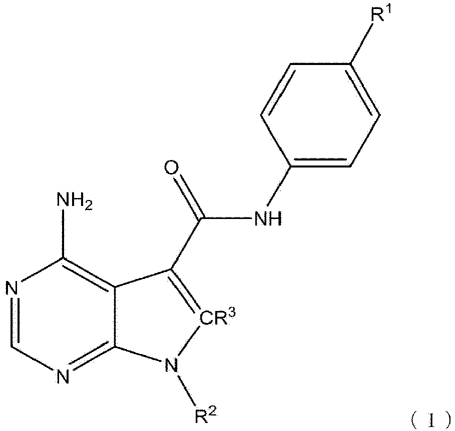 wherein
R¹ is C1-C6 alkoxyalkyl,
R² is substituted or unsubstituted C3-C5 cycloalkyl, and
R³ is hydrogen,
substituted or unsubstituted C2-C6 alkynyl, or
substituted or unsubstituted C1-C6 alkoxy.
